# EUROPEAN PATENT SPECIFICATION

(11) **EP 1 210 347 B1**
(45) Date of publication and mention of the grant of the patent: **23.06.2004**
(21) Application number: 00965970.7
(22) Date of filing: 08.09.2000
(51) Int. Cl.: C07D 473/32, C07D 473/18, C07D 473/34, C07D 239/46, C07C 43/188, C07C 35/18, C07F 7/18, A61K 31/52, A61K 31/522, A61P 31/12

(54) **CARBOCYCLIC NUCLEOSIDES AND PROCESS FOR OBTAINING SUCH**
CARBOCYCLISCHE NUKLEOSIDE UND VERFAHREN ZU DEREN HERSTELLUNG
NUCLEOSIDES CARBOCYCLIQUES ET PROCEDE DE PRODUCTION CORRESPONDANT

(30) Priority: 10.09.1999 US 153086 P; 10.09.1999 US 153087 P; 29.06.2000 US 214897 P
(43) Date of publication of application: 05.06.2002
(73) Proprietor: Stichting REGA V.Z.W., 3000 Leuven (BE)
(72) Inventor: HERDEWIJN, Piet, B-3111 Rotselaar (BE); WANG, Jing, B-9100 Sint-Niklaas (BE); DE CLERCQ, Erik, B-3360 Lovenjoel (BE)
(74) Representative: Prins, Hendrik Willem
(86) International application number: PCT/EP2000/008882
(87) International publication number: WO 2001/018003

(56) References cited:
- WO-A-91/15488
- WO-A-93/17020
- WO-A-93/25565
- GB-A- 2 020 655
- GB-A- 2 097 785
- G. H. POSNER ET AL: "Highly stereocontrolledsynthesis of some trioxygenated cyclohexenes: an asymmetric total synthesis of (-)-methyl-triacetyl-4-epishikimate" JOURNAL OF THE AMERICAN CHEMICAL SOCIETY., vol. 108, no. 23, 1986, pages 7373-7377, XP002157745 DC US
- J. WANG ET AL: "Enantioselective synthesis and conformational study of cyclohexene carbocyclic nucleosides" JOURNAL OF ORGANIC CHEMISTRY, vol. 64, no. 21, 15 October 1999 (1999-10-15), pages 7820-7827, XP002157746 EASTON US
- J. WANG ET AL: "The cyclohexene ring system as a furanose mimic: synthesis and antiviral activity of both enantiomers of cyclohexenylguanine" JOURNAL OF MEDICINAL CHEMISTRY, vol. 43, no. 4, 24 February 2000 (2000-02-24), pages 736-745, XP002157747 WASHINGTON US

## Description

The invention relates to carbocyclic nucleoside analogues and their pharmaceutically acceptable salts, and to a method for the production of such, and to their use as anti-viral agents, amongst others.

Most antiviral compounds belong to the nucleoside field and the development of new modified nucleosides as antiviral agents is an active field of research. An object of the present invention is to provide an agent exhibiting pharmaceutical activity.

According to a first aspect of the present invention there is provided a carbocyclic nucleoside analogue as described in claims 1-14.

These carbocyclic nucleotides exhibit good pharmaceutical activity.

According to a second aspect of the present invention there is provided a process for providing these carbocyclic compounds and intermediates thereof according to claims 15-24.

According to a further aspect of the present invention there is provided a carbocyclic compound according to any of the claims 24-35.

According to a further aspect of the present invention there is provided a pharmaceutical composition and the use of such, according to the claims 36-39.

Further aspects of the invention are detailed in claims 40-43.

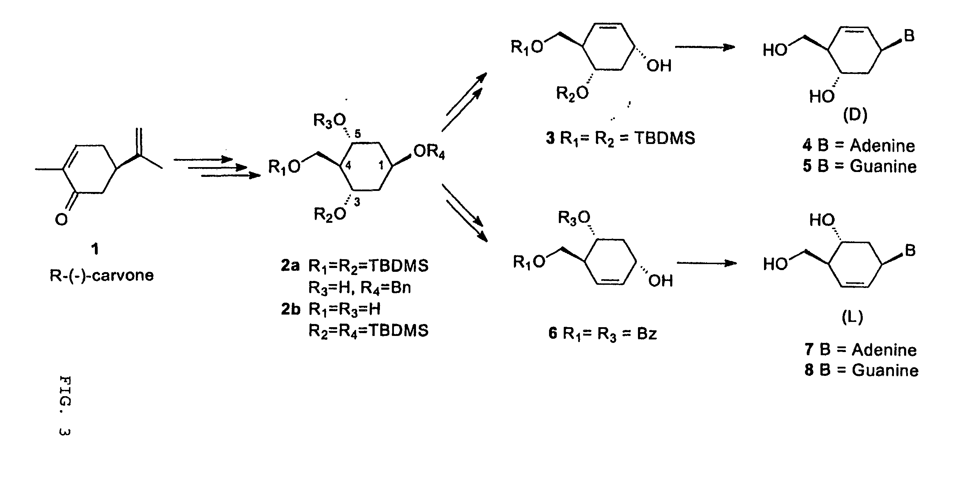

The inventors have developed an enantioselective approach toward the synthesis of (D)-cyclohexene nucleoside **4** (Figure 3) using R-(-)-carvone (1) as inexpensive starting material. A sequence of chemical transformations led to intermediate **2**, possessing four chiral centers and, disregarding the protecting groups R₁-R₄, a plane of symmetry. Intermediate **2** allows for the synthesis of both the (D)- and the (L)-cyclohexene nucleosides **4** and **5**, and **7** and **8**, respectively.

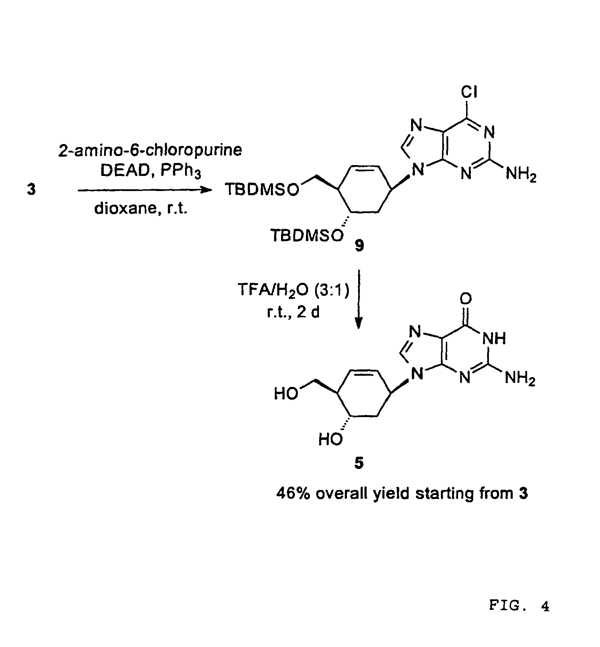

The synthesis of (D)-adenine cyclohexene nucleoside **4** was accomplished by using a Mitsunobu type reaction on enol **3** to introduce the adenine base moiety on the cyclohexenyl ring. The corresponding guanine derivative 5 was synthesized in a similar way. Enol **3** was reacted with 2-amino-6-chloropurine in the presence of DEAD and triphenyl phosphine in dioxane to give **9** (Figure 4), which was converted to **5** by treatment with TFA-H₂O 3:1. Under these reaction conditions the two TBDMS protecting groups were simultaneously removed. The overall yield starting from **3** was 46%. Analytically pure 5 was obtained by reversed-phase HPLC purification.

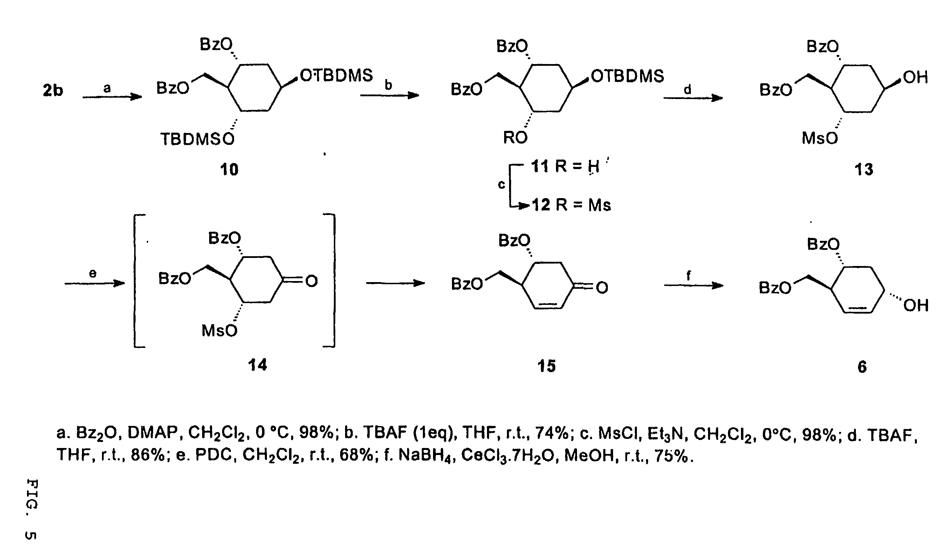

The synthesis of the (L)-cyclohexene nucleosides **7** and **8** was carried out by protection of the C4-CH₂OH and C5-OH groups (2b), followed by conversion of the OR₂ and OR₄ groups into enol **6**. This compound was then used for the introduction of the base moiety according to the same strategy as used for the (D)-series. Intermediate **2b** (R₁ = R₃ = H; R₂ = R₄ = TBDMS) was converted to dibenzoate 10 (Figure 5) using standard reaction conditions (Bz₂O, DMAP, CH₂Cl₂, 98%). The equatorial C3-OTBDMS protecting group was selectively removed in the presence of the axial C1-OTBDMS to give **11** by using one equivalent of TBAF in THF at room temperature (74%). The selectivity of the desilylation reaction has been observed before^{13c}. The C3 alcohol **11** was converted to the corresponding mesylate **12**, the C1-OTBDMS group was removed using TBAF to give the alcohol **13** (96%), which was oxidized using PDC in CH₂Cl₂. This oxidation was accompanied by simultaneous elimination of the C3-OMs group to afford directly the desired enone **15** (68%). Stereoselective reduction of enone **15** using NaBH₄ in the presence of CeCl₃.7H₂O in MeOH gave enol **6** (75%).

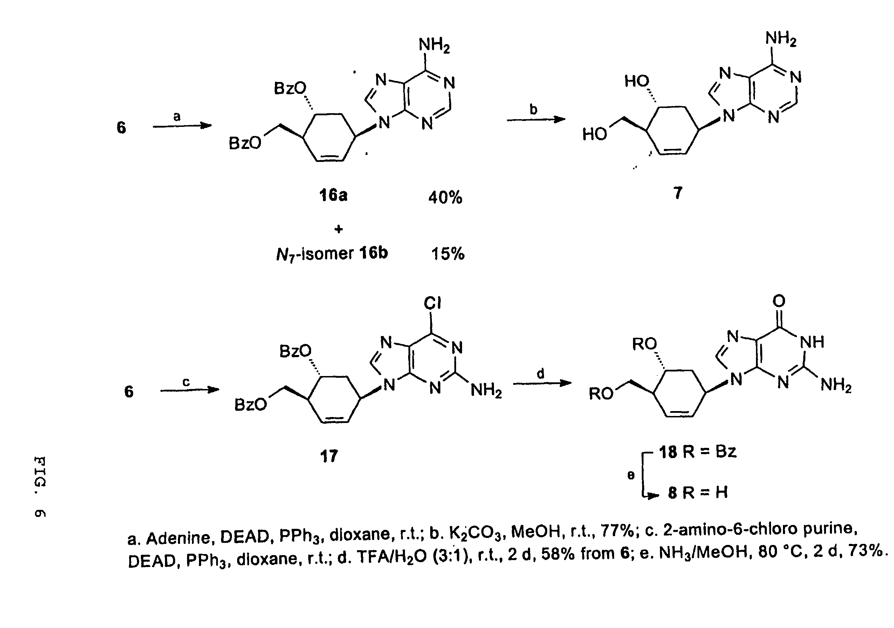

A Mitsunobu reaction was then applied for introduction of the base moiety (Figure 6). Upon reaction of **6** with adenine in the presence of DEAD and PPh₃ in dioxane, the desired adenine derivative **16a** was isolated in 40% yield, together with 15% of N7-isomer **16b** (Figure 6). Finally, removal of the benzoyl protecting groups using K₂CO₃ in MeOH gave the (L)-adenine cyclohexene nucleoside **7** in 72% yield. The corresponding (L)-guanine nucleoside **8** was synthesized in an analogous way. The enol **6** was treated with 2-amino-6-chloropurine under the same reaction condition as described above for **9,** and the obtained 6-chloropurine **17** was converted to the guanine derivative **18** using TFA-H₂O 3:1 (58% yield from **6**). Final deprotection was carried out by heating **18** in a saturated solution of NH₃ in MeOH in a sealed vessel for 2 days and reversed-phase HPLC purification gave pure (L)-guanine cyclohexene nucleoside **8** in 73% yield.

### Antiviral activity

The anti-herpesvirus activity of D-cyclohexene G and L-cyclohexene G and the respective adenine analogues was determined in human embryonic skin muscle fibroblast (E₆SM: HSV-1, HSV-2) and in human embryonic lung (HEL) cells [varicella-zoster virus (VZV), cytomegalovirus (CMV)] (Table I). The source of the viruses and the methodology used to monitor antiviral activity have been previously described (De Clercq, E., Descamps, J., Verhelst, G., Walker, R.T., Jones, A.S., Torrence, P.F., Shugar, D. Comparative efficacy of antiherpes drugs against different strains of herpes simplex virus. J.Infect. Dis. 1980, **141**, 563-574; De Clercq, E., Hóly, A., Rosenberg, I., Sakuma, T., Balzarini, J., Maudgal, P.C. A novel selective broad-spectrum anti-DNA virus agent. Nature, 1986, **323**, 464-467). The antiviral activity was compared with the activity of known and approved antiviral drugs from which two with a purine base moiety (acyclovir, ganciclovir) and two with a pyrimidine base moiety (brivudine, cidofovir).

D-cyclohexene G as well as L-cyclohexene G did not show toxicity in four different cell lines (HeLa, Vero, E₆SM, HEL) (Table II), pointing to their selective antiviral mode of action, as reflected by the high selectivity index of the compounds (Table I). A salient feature is that the activity spectrum of both enantiomers is remarkably similar. Both compounds display the same activity against HSV-1 and HSV-2. Against VZV and CMV the potency of L-cyclohexene G is about 2-fold lower than that of D-cyclohexene G. Against HSV-1, the cyclohexene G nucleosides are as active as acyclovir and brivudin. Against HSV-2, their activity is very similar to that of acyclovir. The cyclohexene G nucleosides retain activity against the TK⁻ strains of HSV-1 and VZV, although the activity is reduced as compared to the activity against the wild type. The activity of D-cyclohexene G against TK⁺ and TK⁻ VZV strains is higher than the respective activities of acyclovir and brivudin against these viruses. D-cyclohexene G has the same activity against CMV as ganciclovir. In conclusion the activity spectrum of the cyclohexene nucleosides of the present invention is very similar to that of the known antiviral compounds possesing a guanine base moiety (acyclovir, ganciclovir). Both the D- and the L-enantiomers of cyclohexene G are antivirally active. The high selectivity indexes observed for D- and L-cyclohexene G indicates the utility of these compounds against herpesvirus infections.

D-cyclohexene G as well as L-cyclohexene G exhibited potent and selective anti-herpes virus (HSV1, HSV2, VZV, CMV) activity. Their activity spectrum is comparable to that of the known antiviral drugs acyclovir and ganciclovir. D- and L-cyclohexene G represent a very potent antiviral nucleosides containing a six-membered carbohydrate mimic. In contrast to the nucleosides with a cyclohexane, pyranose or hexitol ring, the cyclohexene nucleosides have a very flexible conformation. The inventors theorize that this flexibility may be an important structural determinant for their potent antiviral activity.

### Experimental (1)

### General Methods

Melting points were determined in capillary tubes with a Büchi-Tottoli apparatus and are uncorrected. Ultraviolet spectra were recorded with a Philips PU 8740 UV/vis spectrophotometer. ¹H NMR and ¹³C NMR were determined with a 200 MHz Varian Gemini apparatus with tetramethylsilane as internal standard for the ¹H NMR spectra and DMSO-d₆ (39.6 ppm) or CDCl₃ (76.9 ppm) for the ¹³C NMR spectra (s = singlet, d = doublet, dd = double doublet, t = triplet, br s = broad singlet, br d = broad doublet, m = multiplet). Liquid secondary ion mass spectra (LSIMS) with Cs⁺ as primary ion beam were recorded on a Kratos Concept IH (Kratos, Manchester, U.K.) mass spectrometer equipped with a MASPEC2 data system (Mass Spectrometry Services Ltd., Manchester, U.K.). Samples were directly dissolved in glycerol (gly)/thioglycerol (thgly)/*m*-nitrobenzyl alcohol (nba) and the secondary ions accelerated at 7kV. Scans were performed at 10 s/decade from *m*/*z* 1000 down to *m*/*z* 50. Precoated Machery-Nagel Alugram SIL G/UV₂₅₄ plates were used for TLC (in solvent systems: A CH₂Cl₂-MeOH 98:2, B CH₂Cl₂-MeOH 9:1, C CH₂Cl₂-EtOAc 4:1); the spots were examined with UV light and sulfuric acid/anisaldehyde spray. Elemental analyses were done at the University of Konstanz, Germany.

### 9-[(1S,4R,5S)-5-(tert-butyldimethylsilyloxy)-4-(tert-butyldimethylsilyloxymethyl)-2-cyclohexenyl]-2-amino-6-chloropurine (9)

To a mixture of **3** (130 mg, 0.35 mmol), 2-amino-6-chloropurine (119 mg, 0.70 mmol) and PPh₃ (184 mg, 0.70 mmol) in dry dioxane (7 mL) under N₂ at room temperature was added a solution of DEAD (110 µL, 0.70 mmol) in dry dioxane (3 mL) over a period of 1.5 hr. The reaction mixture was stirred at room temperature for two days and concentrated. The residue was chromatographed on silica gel (CH₂Cl₂-MeOH 50:1, then 20:1) to yield crude **9** (170 mg) as a yellow foam: ¹H NMR (CDCl₃) δ -0.10 (s, 3H), -0.04 (s, 3H), 0.07 (s, 6H), 0.82 (s, 9H), 0.90 (s, 9H), 2.04 (t, 2H, *J* = 5.6 Hz), 2.27 (m, 1H), 3.66 (dd, 1H, *J* = 9.9, 5.1 Hz), 3.77 (dd, 1H, *J* = 9.9, 4.4 Hz), 3.98 (m, 1H), 5.21 (m, 1H), 5.43 (s, 2H, NH₂), 5.79 (dm, 1H, *J* = 9.9 Hz), 6.00 (dm, 1H, *J* = 9.9 Hz), 7.79 (s, 1H); ¹³C NMR (CDCl₃) δ -5.6 (q), -5.5 (q), -5.1 (q), -4.8 (q), 17.8 (s), 18.2 (s), 25.6 (q), 25.8 (q), 36.0 (t), 46.9 (d), 49.2 (d), 62.9 (t), 64.6 (d), 124.4 (d), 125.4 (s), 134.4 (d), 141.3 (d), 151.1 (s), 153.3 (s), 159.1 (s);

### 9-[(1S,4R,5S)-5-hydroxy-4-hydroxymethyl-2-cyclohexenyl]guanine (5)

Crude **9** (170 mg) was treated with TFA-H₂O (3:1, 10 mL) at room temperature for two days. The reaction mixture was concentrated and co-evaporated with toluene (2x). The residue was chromatographed on silica gel (CH₂Cl₂-MeOH 10:1, then 1:1) to afford **5** (45 mg, 46% overall yield starting from **3**): Mp >230 °C; UV λₘₐₓ (MeOH) = 253 nm, ¹H NMR (CD₃OD) δ 1.94-2.27 (m, 3H), 3.77 (d, 2H, *J* = 4.7 Hz), 3.85 (m, 1H), 5.17 (m, 1H), 5.88 (dm, 1H, *J* = 10.2 Hz), 6.09 (dm, 1H, *J* = 10.2 Hz), 7.73 (s, 1H); ¹³C NMR (CD₃OD) δ 37.1 (t), 47.7 (d), 50.6 (d), 63.1 (t), 64.8 (d), 125.8 (d), 135.4 (d), 138.5 (d); LISMS (THGLY/NBA) 278 (M+H)⁺; HRMS calcd for C₁₂H₁₆N₅O₃ (M+H)⁺ 278.1253, found 278.1270; Anal. Calcd for C₁₂H₁₅N₅O₃.0.77H₂O: C 49.49, H 5.73, N 24.05; Found: C 49.45, H 5.55, N 24.22.

### (1S,2S,3R,5R)-3-Benzoyl-2-benzoylmethyl-1,5-di(tert-butyldimethylsilyloxy)-cyclohexane (10)

To a solution of **2b** (2.2 g, 5.64 mmol) in dry dichloromethane (20 mL) at 0 °C under N₂ was added DMAP (3.44 g, 28.2 mmol, 5 eq) and Bz₂O (3.83 g, 16.92 mmol, 3 eq) sequentially and in portions. After stirring at 0 °C for 2 hr, the reaction was quenched with ice. The reaction mixture was poured into CH₂Cl₂ (250 ml) and washed with water and brine, dried over Na₂SO₄ and concentrated. The crude product was chromatographed on silica gel (*n*-hexane-EtOAc 10:1) to yield **10** (3.3 g, 98%) as a light yellow oil.
¹H NMR (CDCl₃) δ 0.03 (s, 3H), 0.06 (s, 3H), 0.13 (s, 3H), 0.17 (s, 3H), 0.89 (s, 9H), 1.01 (s, 9H), 1.58 (m, 2H), 2.09 (m, 2H), 2.37 (m, 1H), 4.29 (br-s, 1H), 4.40 (td, 1H, *J* = 10.3, 4.0 Hz), 4.49 (dd, 1H, *J =* 11.4, 2.0 Hz), 4.61 (dd, 1H, *J =* 11.4, 2.2 Hz), 5.66 (td, 1H, *J =* 11.0, 4.6 Hz), 7.37 - 7.44 (m, 4H), 7.49 - 7.59 (m, 2H), 8.02 (m, 4H);
¹³C NMR (CDCl₃) δ -5.3 (q), -5.2 (q), -5.1 (q), -4.5 (q), 17.8 (s), 25.6 (q), 25.7 (q), 38.2 (t), 42.4 (t), 49.5 (d), 60.0 (t), 65.2 (d), 66.4 (d), 68.5 (d), 128.3 (d), 129.6 (d), 130.3 (s), 130.4 (s), 132.8 (d), 165.6 (s), 166.5 (s);

### (1S,2S,3R,5S)-3-Benzoyl-2-benzoylmethyl-5-(tert-butyldimethylsilyloxy)-cyclohexanol (11)

A solution of TBAF 1M in THF (5.38 ml, 5.38 mmol) was added slowly to a solution of **10** (3.23 g, 5.38 mmol) in THF (50 mL) at 0 °C. The reaction mixture was stirred at 0 °C for 2 hr and further at room temperature for 3 hr. Ice was added and the reaction mixture was poured into EtOAc (300 ml) which was washed with NH₄Cl solution, water and brine, dried over Na₂SO₄ and concentrated. The crude product was purified on silica gel (*n*-hexane-EtOAc 5:1 then 1:1) to yield **11** (1.93 g, 74%) as a white foam.
¹H NMR (CDCl₃) δ 0.02 (s, 3H) , 0.09 (s, 3H) , 0.81 (s, 9H) , 1.50 - 1.63 (m, 2H), 1.96 (m, 1H), 2.13 (m, 1H), 2.32 (m, 1H), 3.24 (d, 1H, *J* = 4.8 Hz, -OH), 4.04 (m, 1H), 4.25 (m, 1H), 4.33 (dd, 1H, *J* = 11.4, 2.2 Hz), 5.04 (dd, 1H, *J* = 11.4, 2.2 Hz), 5.60 (td, 1H, *J* = 11.0, 4.5 Hz), 7.36 - 7.60 (m, 6H), 8.06 (m, 4H);
¹³C NMR (CDCl₃) δ -5.2 (q), 17.6 (s), 25.4 (q), 38.2 (t), 40.8 (t), 50.4 (d), 59.8 (t), 64.1 (d), 66.1 (d), 68.4 (d), 128.4 (d), 129.6 (d), 129.8 (d), 130.4 (s), 133.0 (d), 133.2 (d), 165.6 (s), 167.6 (s);
LISMS (THGLY/TFA): 485 (M+H)⁺; HRMS calcd for C₂₇H₃₇O₆Si (M+H)⁺ 485.2359, found 485.2376.

### (1S,2S,3R,5S)-3-Benzoyl-2-benzoylmethyl-5-(tert-butyldimethylsilyloxy)-1-methanesulfonyloxy-cyclohexane (12)

To a solution of **11** (1.90 g, 3.92 mmol) in dry dichloromethane (20 mL) at 0 °C under N₂ was added slowly triethylamine (2.71 ml, 19.6 mmol, 5 eq) and MsCl (456 µl, 5.89 mmol, 1.5 eq) sequentially. After stirring at 0 °C for 1 hr, the reaction was quenched with ice. The reaction mixture was poured into CH₂Cl₂ (250 ml) and washed with a saturated NH₄Cl solution, water and brine, dried over Na₂SO₄ and concentrated. The residue was chromatographed on silica gel (*n*-hexane-EtOAc 1:1) to afford 12 (2.17 g, 98%) as a white foam.
¹H NMR (CDCl₃) δ 0.13 (s, 3H) , 0.15 (s, 3H) , 0.96 (s, 9H) , 1.62 (td, 1H, *J* = 12.0, 2.0 Hz), 1.85 (td, 1H, *J* = 12.0, 2.0 Hz), 2.26 - 2.58 (m, 3H), 2.98 (s, 3H), 4.34 (m, 1H), 4.50 (dd, 1H, *J* = 11.4, 2.0 Hz), 4.60 (dd, 1H, *J* = 11.4, 2.0 Hz), 5.28 (td, 1H, *J =* 11.1, 4.8 Hz), 5.69 (td, 1H, *J* = 11.0, 4.8 Hz), 7.42 (m, 4H), 7.57 (m, 2H), 8.03 (m, 4H);
¹³C NMR (CDCl₃) δ -5.3 (q), -5.2 (q), 17.8 (s), 25.5 (q), 37.8 (t), 38.1 (q), 39.9 (t), 46.7 (d), 58.9 (t), 65.9 (d), 67.8 (d), 75.9 (d), 128.5 (d), 129.6 (d), 129.9 (s), 133.1 (d), 165.4 (s), 166.3 (s);
LISMS (THGLY/GLY): 563 (M+H)⁺; HRMS calcd for C₂₈H₃₉O₈SSi (M+H)⁺563.2135, found 563.2188.

### (1R,3S,4S,5R)-5-Benzoyl-4-benzoylmethyl-3-methanesulfonyloxy-cyclohexanol (13)

To a solution of **12** (2.15 g, 3.82 mmol) in THF (50 mL) at room temperature was added slowly a 1 M solution of TBAF (7.64 ml, 7.64 mmol, 2 eq) in THF. The reaction was stirred at room temperature for 2.5 hr and quenched with ice. After standard work-up and purification on silica gel (*n*-hexane-EtOAc 1:1), **13** (1.55 g, 86%) was obtained as a white foam.
¹H NMR (CDCl₃) δ 1.71 (td, 1H, *J* = 12.1, 2.2 Hz), 1.90 (td, 1H, *J* = 12.2, 2.3 Hz), 2.29 - 2.65 (m, 4H), 3.00 (s, 3H), 4.41 (m, 1H), 4.52 (dd, 1H, *J* = 11.7, 2.8 Hz), 4.61 (dd, 1H, *J* = 11.7, 2.8 Hz), 5.27 (td, 1H, *J* = 10.6, 4.8 Hz), 5.65 (td, 1H, *J* = 10.6, 4.7 Hz), 7.42 (m, 4H), 7.55 (m, 4H), 8.02 (m, 4H);
¹³C NMR (CDCl₃) δ 37.3 (t) , 38.2 (q) , 39.0 (t), 46.8 (d) , 59.3 (t), 65.1 (d), 67.9 (d), 75.8 (d), 128.5 (d), 129.7 (d), 133.2 (d), 133.3 (d), 165.6 (s), 166.4 (s);
LISMS (THGLY/TFA): 449 (M+H)⁺; HUMS calcd for C₂₂H₂₅O₈S (M+H)⁺ 449.1270, found 449.1244.

### (4S,5R)-5-Benzoyl-4-benzoylmethyl-cyclohex-2-en-1-one (15)

A mixture of **13** (500 mg, 1.12 mmol) and PDC (2.1 g, 5.60 mmol, 5 eq) in dry CH₂Cl₂ (30 mL) was stirred vigorously at room temperature for 24 h. The reaction mixture was filtered through Celite® and washed with CH₂Cl₂. The filtrate was concentrated and the residue was chromatoghraphed on silica gel (*n*-hexane-EtOAc 2:1, then 1:2) to yield starting material **13** (100 mg, 20 %) and enone 15 (267 mg, 68%) as a light yellow oil.
¹H NMR (CDCl₃) δ 2.73 (dd, 1H, *J* = 16.5, 8.8 Hz), 3.10 (dd, 1H, *J* = 16.5, 4.4 Hz), 3.27 (m, 1H), 4.50 (dd, 1H, *J* = 11.3, 4.7 Hz), 4.66 (dd, 1H, *J* = 11.3, 5.5 Hz), 5.66 (ddd, 1H, *J* = 8.8, 7.3, 4.4 Hz), 6.26 (dd, 1H, *J* = 10.2, 2.2 Hz), 6.96 (dd, 1H, *J* = 10.2, 3.3 Hz), 7.40 - 7.63 (m, 6H), 8.01 (m, 4H);
¹³C NMR (CDCl₃) δ 41.3 (d), 42.1 (t), 63.2 (t), 70.0 (d), 128.6 (d), 129.5 (2s), 129.7 (d), 129.8 (d), 131.4 (d), 133.5 (d), 146.5 (d), 165.5 (s), 166.4 (s), 195.8 (s);

### (1S,4S,5R)-5-Benzoyl-4-benzoylmethyl-cyclohex-2-en-1-ol (6)

To a solution of **15** (267 mg, 0.76 mmol) in MeOH (10 mL) at room temperature under N₂ was added CeCl₃.7H₂O (426 mg, 1.14 mmol, 1.5 eq). The mixture was stirred for 0.5 h and a clear solution was obtained. NaBH₄ (35 mg, 0.91 mmol, 1.2 eq) was added in portions and H₂ evolved. The reaction mixture was stirred for 1 h and quenched with ice. The mixture was stirred for 15 min and concentrated. The residue was distributed into EtOAc, washed with H₂O and brine, dried over Na₂SO₄ and concentrated. The residue was chromatographed on silica gel (*n*-hexane-EtOAc 10:1) to give **6** (200 mg, 75%) as a light yellow oil.
¹H NMR (CDCl₃) δ 1.77 (d, 1H, *J* = 7.2 Hz), 1.93 (ddd, 1H, *J* = 12.1, 10.2, 8.0 Hz), 2.54 (ddd, 1H, *J* = 12.1, 5.8, 3.3 Hz), 3.00 (m, 1H), 4.32 (dd, 1H, *J* = 11.4, 5.5 Hz), 4.44 (dd, 1H, *J* = 11.4, 5.5 Hz), 9.50 (m, 1H), 5.35 (ddd, 1H, *J* = 10.2, 7.3, 2.9 Hz), 5.78 (dt, 1H, *J* = 10.2, 1.8 Hz), 5.97 (dt, 1H, *J* = 10.2, 2.5 Hz), 7.34 - 7.60 (m, 6H), 8.00 (m, 4H);
¹³C NMR (CDCl₃) δ 36.6 (t), 40.9 (d), 46.6 (t), 65.8 (d), 69.9 (d), 126.6 (d), 128.4 (d), 128.5 (d), 129.7 (d), 129.8 (s), 130.9 (s), 132.7 (d), 133.1 (d), 133.2 (d), 166.0 (s), 166.5 (s);
LISMS (THGLY/TFA): 353 (M+H)⁺; HUMS calcd for C₂₁H₂₁O₅ (M+H)⁺ 353.1389, found 353.1440.

### 9-[(1R,4S,5R)-5-Benzoyl-4-benzoylmethyl-2-cyclohexenyl]adenine (16a)

To a mixture of **6** (65 mg, 0.18 mmol), adenine (48 mg, 0.36 mmol, 2 eq) and PPh₃ (94 mg, 0.36 mmol, 2 eq) in dry dioxane (4 mL) under N₂ at room temperature was added a solution of DEAD (56 µL, 0.36 mmol, 2 eq) in dry dioxane (3 mL) over a period of 1 hr. The reaction mixture was stirred at room temperature overnight and concentrated. The residue was chromatographed on silica gel (CH₂Cl₂-MeOH 50:1, 20:1, 10:1) to yield **16a** (33 mg, 40%) as a white solid.
UV λₘₐₓ (MeOH): 231 and 263 nm.
¹H NMR (CDCl₃) δ 2.48 (ddd, 1H, *J* = 13.6, 8.3, 5.8 Hz), 2.57 (ddd, 1H, *J* = 13.6, 6.0, 3.2 Hz), 4.50 (dd, 1H, *J* = 10.4, 5.0 Hz), 4.63 (dd, 1H, J = 10.4, 6.1 Hz), 5.53 (m, = 2H), 5.92 (s, 2H), 6.09 (dm, 1H, *J*= 10.0 Hz), 6.17 (dm, 1H, *J* = 10.0 Hz), 7.41 (m, 4H), 7.57 (m, 2H), 7.86 (s, 1H), 8.04 (m, 4H), 8.35 (s, 1H);
¹³C NMR (CDCl₃) δ 32.4 (t), 40.6 (d), 48.7 (d), 64.3 (t), 68.2 (d), 120.1 (s), 126.8 (d), 128.5 (d), 128.6 (d), 129.6 (d), 129.7 (d), 131.0 (d), 133.4 (d), 138.8 (d), 149.8 (s), 153.1 (d), 155.8 (s), 165.8 (s), 166.5 (s); LISMS (THGLY/NBA): 470 (M+H)⁺; HRMS calcd for C₂₆H₂₄N₅O₄ (M+H)⁺ 470.1828, found 470.1845.

### 9-[(1R,4S,5R)-5-hydroxy-4-hydroxymethyl-2-cyclohexenyl]adenine (7)

Compound **16a** (33 mg, 0.07 mmol) was treated with anhydrous K₂CO₃ (100 mg) in MeOH (3 mL) at room temperature for 3 hr. Small portion of silica gel was added to the reaction mixture and the solvent was evaporated. The residue was chromatographed on silica gel (CH₂Cl₂-MeOH 10:1, 1:1) to give **7** (14 mg, 77%).
¹H NMR (CD₃OD) δ 2.02 - 2.32 (m, 3H), 3.79 - 3.90 (m, 3H), 5.35 (m, 1H), 5.93 (dm, 1H, *J* = 9.9 Hz), 6.15 (dm, 1H, *J* = 9.9 Hz), 8.09 (s, 1H), 8.21 (s, 1H);
¹³C NMR (CD₃OD) δ 37.2 (t), 47.7 (d), 51.1 (d) , 63.0 (t), 64.7 (d), 120.6 (s), 125.4 (d), 136.0 (d), 141.6 (d), 150.3 (s), 153.8 (d), 157.5 (s);
LISMS (THGLY/TFA): 262 (M+H)⁺; HRMS calcd for C₁₂H₁₆N₅O₂ (M+H)⁺ 262.1304, found 262.1323.

### 9-[(1R,4S,5R)-5-Benzoyl-4-benzoylmethyl-2-cyclohexenyl]guanine (18)

Compound **6** (160 mg, 0.45 mmol) was treated with 2-amino-6-chloropurine (153 mg, 0.90 mmol, 2 eq) in the presence of PPh₃ (235 mg, 0.90 mmol, 2 eq) and DEAD (140 µl, 0.90 mmol, 2 eq) in dry dioxane (12 mL) at room temperature overnight. After concentration and purification on silica gel (CH₂Cl₂-EtOAc 1:1), crude **17** (500 mg) was obtained, which was treated with CF₃COOH/H₂O (3:1, 12 mL) at room temperature for 2 days. The reaction mixture was concentrated and coevaporated with toluene. The residue was purified on silica gel (CH₂Cl₂-MeOH 20:1) to yield **18** (126 mg, 58% over two steps) as a white solid.
UV λₘₐₓ (MeOH): 251 and 256 nm.
¹H NMR (500 MHz, DMSO-d₆) δ 2.30 (ddd, 1H, *J* = 13.6, 8.3, 5.9 Hz), 2.42 (ddd, 1H, *J* = 13.6, 6.4, 3.2 Hz), 3.00 (m, 1H), 4.52 (m, 2H), 5.17 (m, 1H), 5.37 (m, 1H), 6.03 (dm, 1H, *J* = 10.2 Hz), 6.11 (dm, 1H, *J* = 10.2 Hz), 6.45 (s, 2H), 7.51 (m, 4H), 7.66 (m, 2H), 7.69 (s, 1H), 7.95 (m, 4H), 10.61 (s, 1H);
¹³C NMR (DMSO-*d*₆) δ 31.4 (t), 40.0 (d, overlapped with DMSO-*d*₆ peak), 47.9 (d), 64.4 (t), 68.5 (d), 116.9 (s), 127.0 (d), 128.9 (d), 129.3 (d), 129.4 (d), 130.2 (d), 133.6 (d), 135.7 (d), 150.9 (s), 153.8 (s), 156.9 (s), 165.3 (s), 165.8 (s);
LISMS (THGLY/GLY): 486 (M+H)⁺; HRMS calcd for C₂₆H₂₄N₅O₅ (M+H)⁺ 486.1777, found 486.1816;
UV (MeOH): 231, 256.

### 9-[(1R,4S,5R)-5-Hydroxy-4-hydroxymethyl-2-cyclohexenyl]guanine (8)

A mixture of **18** (85 mg) in an ammonium MeOH solution (75 mL) was sealed and heated at 80 °C for 2 days. After cooling to room temperature, the mixture was concentrated and the residue was purified by reverse *HPLC* (4% CH₃CN in water) to afford **8** (36 mg, 75%) as a white powder.
Mp: 255 °C (decomp.)
UV λₘₐₓ (MeOH) = 254 nm.
¹H NMR (500 HMz, DMSO-*d*₆) δ 1.85 (m, 1H), 1.98 (m, 1H), 2.11 (m, 1H), 3.54 (dd, 1H, J = 10.3, 5.5 Hz), 3.60 (dd, 1H, J = 10.3, 4.8 Hz), 3.70 (m, 1H), 4.68 (br-s, 1H, -OH), 4.75 (br-s, 1H, -OH), 4.99 (m, 1H), 5.77 (dm, 1H, *J* = 9.8 Hz), 5.97 (dm, 1H, *J* = 9.8 Hz), 6.57 (s, 2H, -NH₂), 7.50 (s, 1H), 10.8 (br-s, 1H, -NH);
¹³C NMR (125 MHz, DMSO-*d*₆) δ 35.9 (t), 46.4 (d), 48.2 (d), 61.5 (t), 62.7 (d), 116.9 (s), 124.8 (d), 133.7 (d), 135.6 (d), 150.8 (s), 154.1 (s), 157.6 (s);
LISMS (THGLY/NBA) 278 (M+H)⁺; HRMS calcd for C₁₂H₁₆N₅O₃ (M+H)⁺ 278.1253, found 278.1247; Anal. Calcd for C₁₂H₁₅N₅O₃.1.5H₂O: C 47.35, H 5.96, N 23.03; Found: C 47.46, H 5.64, N 22.87.

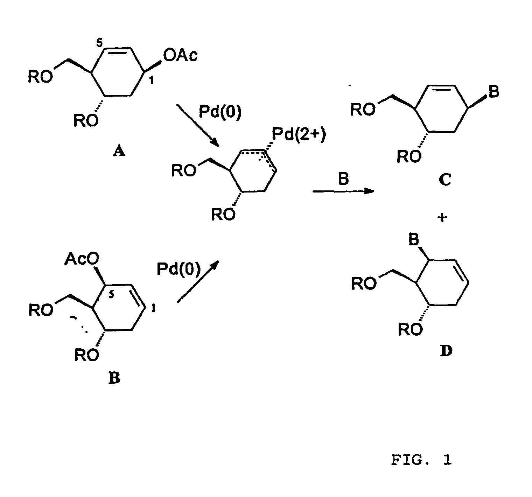

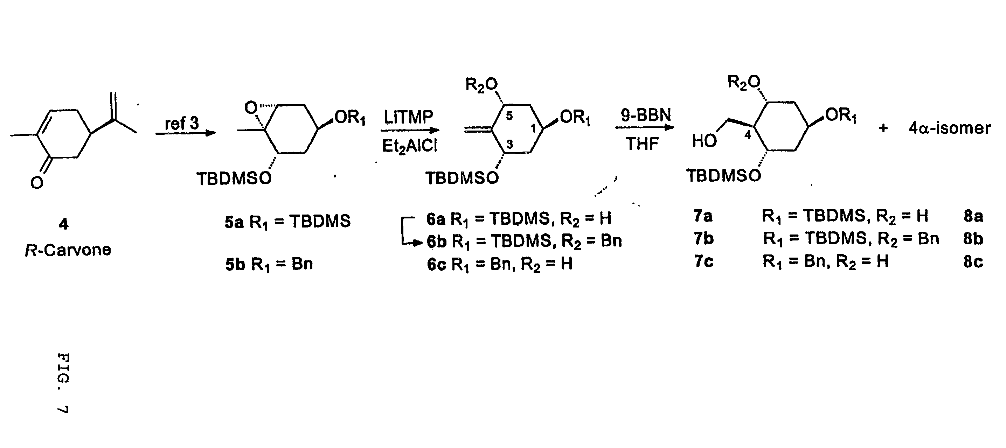

As detailed above, the inventors have developed an enantioselective approach to the synthesis of six-membered carbocyclic nucleosides of type **2b** (R = OH) starting from (R)-(-)-carvone (**4**, Figure 7, corresponding substantially with Figure 1). A key step involving hydroboration of the exo double bond of cyclohexene **6b** to afford hydroxymethyl substituted **7b** with the correct stereochemistry at C4. Precursor **6a** provided an ideal starting material for the synthesis of **3** as it had (1) a protected hydroxyl group at C3, (2) a protected hydroxyl substituent at C1, which at a final stage can be used to introduce a base moiety with retention of the configuration using Pd-chemistry, and (3) a free hydroxyl group at C5, which could be used to introduce the double bond.

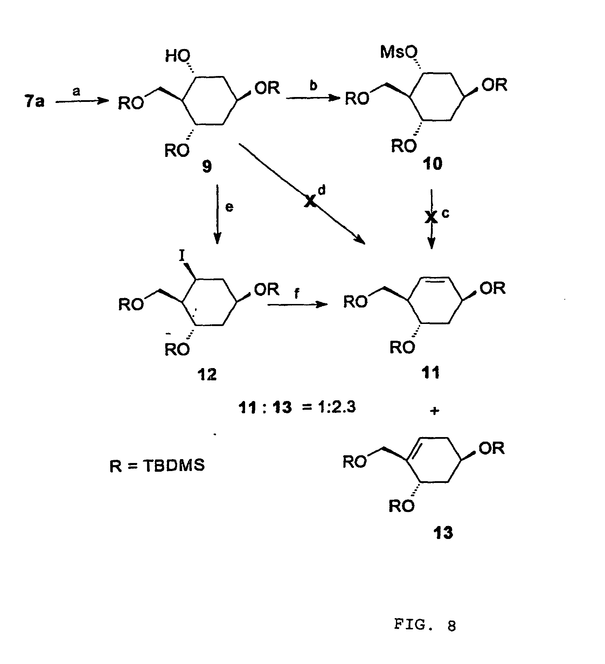

The most straightforward approach seemed to introduce the C5-C6 double bond via conversion of the OH at C5 into a suitable leaving group, followed by a regioselective elimination. The latter might be achieved *via* a E₂-type elimination reaction by treatment with base, which requires a neighbouring hydrogen trans to the leaving group, only available on C6. In order to explore this strategy, alcohol **6a** was converted into diol **7a** *via* hydroboration using 9-BBN in THF. The reaction gave **7a** as the major isomer, together with a small amount of epimer **8a.** The β-stereochemistry at C4 was easily established by NMR spectrometry. Selective protection of the primary hydroxyl group of **7a** (TBDMSCl, imidazole, DMF) gave **9** (Figure 8) and the leaving group was introduced (MsCl, Et₃N, dichloromethane) to give **10**. However, upon treatment of mesylate **10** with DBU in toluene, cyclohexene **11** was not formed. More vigorous reaction conditions (KOH, H₂O-THF),⁵ likewise, failed to yield the unsaturated compound **11**. Direct elimination of the 5-OH of **9** under Mitsunobu conditions (DEAD, PPh₃, THF)⁶ was also unsuccessful. **9** was converted into the β-iodide **12** (I₂, PPh₃, imidazole, toluene), with inversion of the stereochemistry at C5, followed by treatment with DBU in refluxing toluene. This reaction resulted in an inseparable mixture (yield 68%) of cyclohexenes **11** and **13** in a 1:2.3 ratio, respectively, in favour of the undesired regioisomer.

The inventors also investigated a different synthetic strategy, i.e. the construction of an allylic acetate of type **A** or **B** (Figure 1) as intermediate for the Pd coupling reaction to introduce the base moiety.

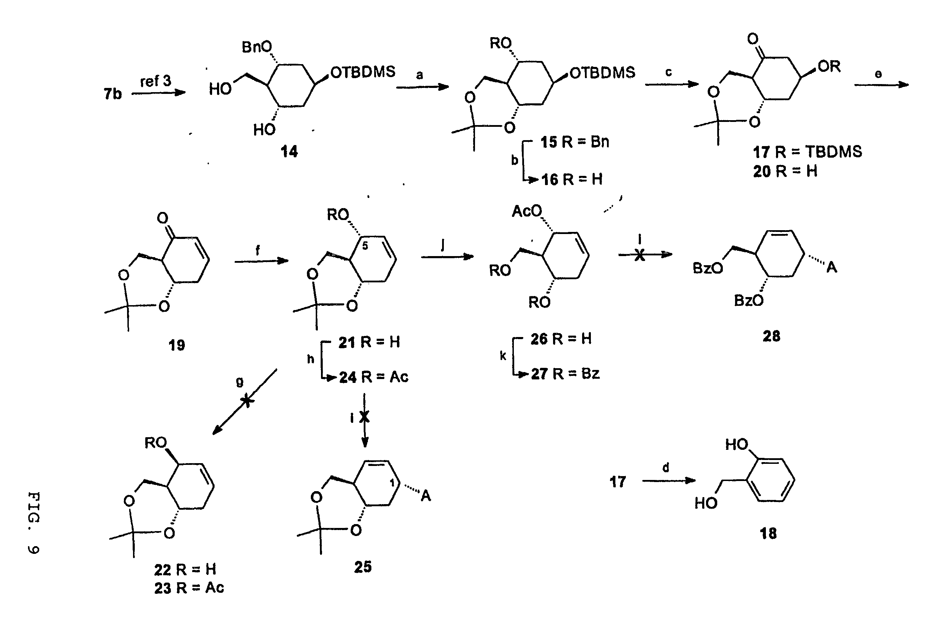

Diol **14** (Figure 9) was protected as cyclic acetal **15** (2,2-dimethoxypropane, PPTS, acetone-THF), the Bn group was removed (10% Pd on carbon, HCOONH₄, MeOH, reflux) to give alcohol **16**, and oxidation of the C5-OH (PDC, dichloromethane) provided ketone **17**. Cleavage of the TBDMS ether using tetrabutylammonium fluoride (TBAF) in THF led mainly to diol **18.** However, under neutral reaction conditions (KF, 18-crown-6, THF) the desired enone **19** was isolated in 62% yield; the β-hydroxy ketone intermediate **20** could not be detected. The critical reduction of enone **19** to the corresponding allylic alcohol **22** with β-oriented ON at C5 proved to be problematic, leading almost exclusively to the α-isomer **21** under the applied reaction conditions (NaBH₄, CeCl₃.7H₂O, MeOH and 9-BBN, THF). In an attempt to invert the stereochemistry at C5 of α-alcohol **21**, the latter was subjected to a Mitsunobu type reaction (DEAD, PPh₃, AcOH), but the desired β-acetate **23** was not formed. However, compound **21** might be used to synthesize the α-analogue of the aforementioned cyclohexene nucleoside, interesting for as well conformational analysis as for determination of antiviral activity.

The intended Pd coupling reaction was investigated on the α-acetate **24**, easily prepared from **21** (Ac₂O, DMAP, dichloromethane). When **24** was treated with the anion (NaH) of adenine in the presence of tetrakis(triphenylphosphine)palladium(0) in DMF-THF, only **24** was recovered and no trace of the 1α-adenine **25** could be detected. Reasoning that this failure might be due to the rigidity of the cyclic acetal present, **24** was treated with PPTS in MeOH to give diol **26**, which was then converted into the corresponding dibenzoate **27** (Bz₂O, DMAP, dichloromethane). However, upon subjection of **27** to the same reaction conditions for coupling as applied above to **24**, the expected 1α-adenine product **28** could not be isolated.

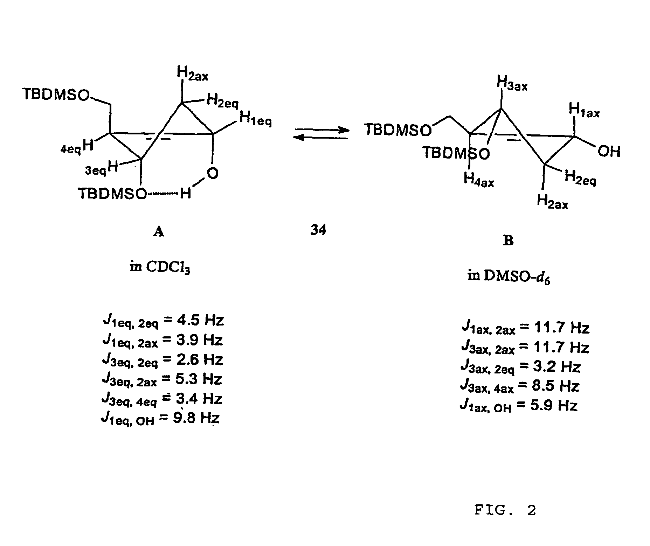

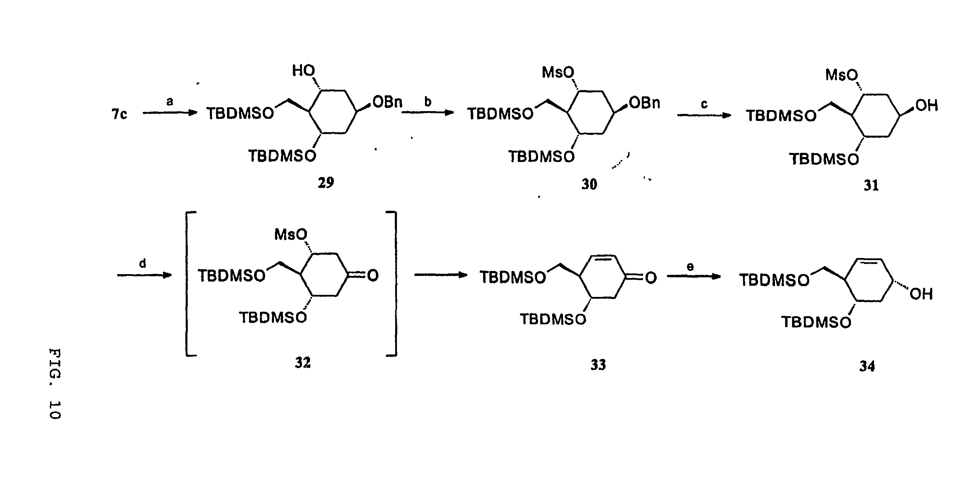

The above failure having exhausted the possibilities of the Pd coupling strategy, the most reliable alternative (for the introduction of the base moiety seemed) a Mitsunobu reaction was utilized, i.e. by substitution with inversion of the configuration of an α-oriented hydroxyl group at C1. Therefore the inventors had to synthesize an appropriately protected precursor **7c**. Epoxide **5b** (Figure 7, R₁ = Bn) was converted into **6c** under the reported conditions (LiTMP and Et₂AlCl in benzene-toluene 1:1) in 79% yield. Hydroboration of **6c** with 9-BBN in THF afforded **7c** as major isomer (74%), together with its epimer **8c** (20%). Similar to configurational assignment of **7a** and **7b**, the β-stereochemistry at C4 of **7c** was established by ¹H-NMR. The primary hydroxyl group of **7c** was selectively protected using 1.2 equivalents of TBDMSC1 and imidazole in DMF t_e 2ve **29** (70%, Figure 10), followed by conversion of the free alcohol at C5 into the corresponding mesylate **30** by treatment with MsCl and Et₃N in dichloromethane. Hydrogenolytic cleavage of the benzyl ether at C1 using 20% Pd(OH)₂ on carbon in the presence of cyclohexene in MeOH gave **31** in low yield (21%), which could be improved to 76% by the use of 10% Pd on carbon and HCOONH₄ in refluxing MeOH. Oxidation of alcohol **31** using PDC in dichloromethane gave a mixture of ketone **32** and enone **33** in a combined yield of 39%. However, using MnO₂ in dichloromethane, an incomplete but clean reaction took place. The ketone **32** was not isolated and enone **33** was obtained in 48% yield and recovered **31** (47%) could be recycled. Finally, enone **33** was reduced using NaBH₄ in the presence of CeCl₃.7H₂O in MeOH and gave the desired α-alcohol **34** as a single isomer in almost quantitative yield. The stereochemistry of **34** was confirmed by ¹H NMR spectral data. In CDCl₃ conformation **A** (Figure 2), with the three substituents in a pseudoaxial position, predominates due to intramolecular hydrogen bonding between the C1-OH and C3-OTBDMS groups, while in DMSO-*d*₆ it adopts conformation **B**. This reflects the much lower axial-equatorial energy differences in cyclohexenes as compared to the corresponding cyclohexanes.

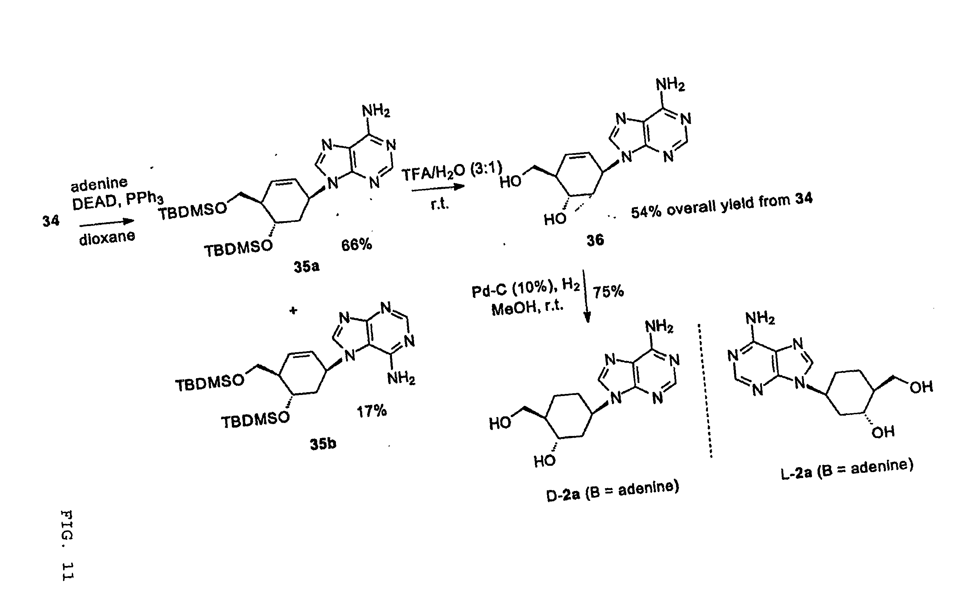

With intermediate **34** in hand, the base moiety (adenine) was introduced under Mitsunobu reaction conditions. Upon treatment of **34** with adenine in the presence of DEAD and PPh₃ in dioxane at room temperature for 1 day, **35a** was isolated in 66% yield, together with 17% of its *N*₇-isomer **35b** (Figure 11). Complete deprotection of **35a** using TRAP in THF at room temperature afforded the desired cyclohexene carbocyclic nucleoside **36** in almost quantitative yield. However, the compound was contaminated with tetrabutylammonium salts which could not be removed by standard chromatographic techniques. Recently Parlow et al. described a work-up procedure to remove tetrabutylammonium salts by the direct addition to the reaction mixture of mixed ionexchange resins Amberlite® 15 and Amberlite® 15 in the Ca²⁺ form. Applied to the above TBAF reaction a complex mixture was obtained, giving **36** in low yield. In order to avoid the use of TBAF, Megron's method (Megron, G.; Vasquezy, F.; Galderon, G.; Cruz, R.; Gavino, R.; Islas, G. Synth. Commun. 1998, 26(16), 3021-3027) was used: compound **35a** was treated with potassium *tert*-butoxide in DMF at room temperature. However, only a complex, reaction mixture was obtained, due to the strong basic character of the reaction conditions. Finally **35a** was treated with a 3:1 mixture of TFA and H₂O at room temperature, which smoothly gave **36** in 54% overall yield starting from **34.** According to our experience, this is the best procedure to cleave TBDMS ethers of this type of compound. Finally, **36** was purified by reversed-phase HPLC for analysis and determination of biological activity.

The above intermediate 36 (Figure 11) gave the inventors the opportunity to obtain as yet 2a (B = adenine) in enantiopure form via reduction of the double bond. Thus, 36 was hydrogenated using H2 under atmospheric pressure in the presence 10% Pd on carbon in MeOH at room temperature to afford D-2a in 75% yield. The spectral data of D-2a were superimposable with those of a DL mixture of 2a. The enantiomeric purity of D-2a was examined by HPLC on a chiral column. The separation of a DL mixture of 2a together with the HPLC profile of D-2a synthesized by the above approach is depicted in reference 3c. Its enantiomeric purity proved 99%, at the same time establishing the high enantiomeric purity of **36.**

The inventors have developed an enantioselective approach towards the synthesis of cyclohexene carbocyclic nucleosides starting from (*R*)-carvone **4**. The synthetic methodology makes use of a Mitsunobu reaction as the key step to introduce the heterocyclic base moiety. The reaction proved to be efficient as well as chemo- and stereoselective, while the commonly applied palladium-mediated coupling strategy was unsuccessful. ¹H NMR and computation results show that in solution the synthesized adenine derivative **36** exists predominantly in a ³H₂ half-chair conformation with the adenine base orienting in a pseudoaxial position. The energy difference between ³H₂ and ²H₃ is, however, low. This compound may therefore be considered as a good mimic of a furanose nucleoside, showing two low energy conformations with a preference for the "3'-endo conformation". This is also the preferred conformation of a hexitol nucleoside, in the ¹C₄ conformation. Moreover, the inventors theorize that the easy interconversion among both conformers might be a factor for antiviral activity.

### Experimental (2)

### (1R,3S,5R)-5-Benzyloxy-3-(tert-butyldimethylsilyloxy)-2-methylenecyclohexanol (6c)

A solution of 2,2,6,6-tetramethylpiperidine (TMP, 27.3 mL, 162 mmol) in dry benzene (80 mL) and dry toluene (80 mL) was cooled to 0 °C under N₂ and a solution of n-BuLi in hexane (1.6 M, 64.8 mL, 162 mmol) was added dropwise. The resulting mixture was stirred at 0 °C for 10 min and a solution of Et₂AlCl (1.8 M, 90 mL, 162 mmol) in toluene was slowly added over a period of 1 hr. The reaction was stirred for an additional 30 min. A solution of **5b** (14.1 g, 40.5 mmol) in benzene (30 mL) was added slowly. The reaction mixture was stirred at 0 °C for 3 h, then poured into an ice-cold NH₄Cl solution (300 mL). A 3 N HCl solution was added until a clear emulsion was obtained. The layers were separated and the aqueous layer was extracted with EtOAc (3x). The combined organic layers were washed with H₂O and brine, dried over Na₂SO₄ and concentrated. The residue was chromatographed on silica gel (*n*-hexane-EtOAc 10:1) to give **6c** (10.2 g, 71%) as a light-yellow oil: ¹H NMR (CDCl₃) δ 0.09 (s, 6H), 0.92 (s, 9H), 1.90 (m, 4H), 2.69 (d, 1H, *J* = 7.3 Hz, OH), 4.05 (m, 1H), 4.45 (m, 2H), 4.58 (s, 2H), 5.05 (s, 1H), 5.07 (s, 1H), 7.33 (m, 5H); ¹³C NMR (CDCl₃) δ -5.1 (q), 18.0 (s), 25.7 (q), 40.7 (t), 40.9 (t), 70.4 (d and t, overlapped), 70.8 (d), 71.3 (d), 107.1 (t), 127.5 (d), 128.4 (d), 138.7 (s), 150.7 (s).

### (1R,2S,3S,5R)-5-Benzyloxy-3-(tert-butyldimethylsilyloxy)-2-hydroxymethyl-cyclohexanol (7c) and its epimer 8c

To a solution of _0 (10.8 g, 31.03 mmol) in dry THF (80 mL) at 0 °C under N₂ was added slowly a solution of 9-BBN in THF (0.5 M, 155 mL, 77.58 mmol). The reaction mixture was slowly warmed up to rt overnight. The reaction was cooled to 0 °C and treated sequentially with EtOH (30 mL), a 2 N NaOH solution (60 mL) and a 35% H₂O₂ solution (60 mL) under stirring. The resulting mixture was stirred at rt for 24 h, then poured into a mixture of EtOAc (300 mL) and H₂O (300 mL). The layers were separated and the aqueous layer was extracted with EtOAc (3x). The combined organic layers were washed with H₂O and brine, dried over Na₂SO₄ and concentrated. The crude product was separated on silica gel (*n*-hexane-EtOAc 5:1, then 1:1) to yield **7c** (8.4 g, 74%) and epimer **8c** (2.28 g, 20%) as a light-yellow oils.
**7c**: ¹H NMR (500 MHz, CDCl₃) δ 0.09 (2s, 6H), 0.91 (s, 9H), 1.52 (ddd, 1H, *J =* 13.1, 10.1, 2.8 Hz), 1.54 (ddd, 1H, *J* = 13.1, 10.1, 3.1 Hz), 1.69 (tdd, 1H, *J* = 10.0, 7.5, 4.1 Hz), 2.10 (dt, 1H, *J* = 13.1, 4.1 Hz), 2.16 (dt, 1H, *J* = 13.1, 4.1 Hz), 2.71 (s, 1H), 3.11 (s, 1H), 3.78 (dd, 1H, *J* = 10.1, 7.5 Hz), 3.85 (td, 1H, *J =* 10.0, 4.2 Hz), 3.86 (m, 1H), 3.97 (br-td, 1H, *J =* 10.1, 4.1 Hz), 4.04 (br-dd, 1H, *J* = 10.1, 4.1 Hz), 4.51 (s, 2H), 7.26-7.37 (m, 5H);
¹³C NMR (CDCl₃) δ -5.0 (q), -4.3 (q), 17.8 (s), 25.7 (q), 38.1 (t), 38.4 (t), 53.2 (d), 63.4 (t), 68.0 (d), 69.4 (d), 70.3 (t), 72.4 (d), 127.4 (d), 127.6 (d), 128.4 (d), 138.7 (s); LISMS (THGLY): 367 (M+H)⁺; HRMS calcd for C₂₀H₃₅O₄Si (M+H)⁺ 367.2305, found 367.2341.
**8c**: ¹H NMR (CDCl₃) δ 0.07 (s, 3H), 0.08 (s, 3H), 0.85 (s, 9H), 1.40-1.87 (m, 3H), 2.25 (dm, 1H, *J* = 13.2 Hz), 2.48 (dm, 1H, *J* = 13.2 Hz), 3.69 - 4.20 (m, 6H), 4.33 (m, 1H), 4.53 (d, 1H, *J* = 11.7 Hz), 4.62 (d, 1H, *J* = 11.7 Hz), 7.33 (m, 5H), 8.79 (s, 1H); ¹³C NMR (CDCl₃) δ -5.6 (q), -5.0 (q), 21.9 (s), 25.5 (q), 39.2 (2t, overlapped), 45.9 (d), 61.3 (t), 69.0 (d), 69.4 (d), 70.4 (t), 70.8 (d), 127.6 (d), 127.7 (d), 128.4 (d), 138.6 (s); LISMS (THYLY): 367 (M+H)⁺; HRMS calcd for C₂₀H₃₅O₄Si (M+H)⁺ 367.2305, found 367.2335.

### (1R,2R,3S,5S)-5-Benzyloxy-3- (tert-butyldimethylsilyloxy)-2-(tert-butyldimethylsilyloxy methyl)cyclohexanol (29).

To a solution of **7c** (2.5 g, 6.83 mmol) in DMF (50 mL) at rt were added imidazole (930 mg, 13.66 mmol) and TBDMSCl (1.23 g, 8.2 mmol) in portions. The reaction was stirred at rt overnight and quenched with ice. The resulting mixture was evaporated to remove DMF and the residue was partitioned between EtOAc and H₂O. The layers were separated and the aqueous layer was extracted with EtOAc (2x). The combined organic layers were washed with H₂O and brine, dried over Na₂SO₄ and concentrated. The residue was chromatographed on silica gel (*n*-hexane-EtOAc 5:1) to yield **29** (2.28 g, 70%) as a light-yellow oil: ¹H NMR (CDCl₃) δ 0.05, 0.06, 0.09 (3s, 12H), 0.89, 0.91 (2s, 18H), 1.53 (m, 2H), 1.72 (qd, 1H, *J* = 9.5, 4.4 Hz), 2.11 (m, 2H), 3.67 (t, 1H, *J* = 9.5 Hz), 3.78 (td, 1H, *J* = 9.5, 4.4 Hz), 3.87 (m, 1H), 4.01 (m, 1H), 4.16 (dd, 1H, *J* = 9.5, 4.4 Hz), 4.46 (d, 1H, *J* = 15.2 Hz), 4.48 (d, 1H, *J* = 15.2 Hz), 7.33 (m, 5H); ¹³C NMR (CDCl₃) δ -5.7 (q), -5.1 (q), -4.3 (q), 17.8, 18.0 (2s), 25.7 (2q), 37.0 (t), 38.4 (t), 52.2 (d), 66.2 (t), 67.2 (d), 70.1 (t and d overlapped), 72.4 (d), 127.3 (d), 127.4 (d), 128.4 (d), 138.9 (s); LISMS (GLY): 481 (M+H)⁺; HRMS cald for C₂₆H₄₉O₄Si₂ (M+H)⁺ 481.3169, found 481.3199.

### (1R,2R,3S,5S)-5-Benzyloxy-2-(tert butyldimethylsilyloxymethyl)-3-(tert-butyldimethylsilyoxy)-1-methanesulfonyloxy-cyclohexane (30)

To a solution of **29** (5.4 g, 11.25 mmol) in CH₂Cl₂ (120 mL) at 0 °C was added triethylamine (7.8 mL, 56.25 mmol), followed by dropwise addition of MsCl (1.3 mL, 16.87 mmol). The reaction was stirred at 0 °C for 1h and treated with ice. The resulting mixture was separated and the aqueous layer was extracted with CH₂Cl₂ (2x). The combined organic layers were washed with a diluted HCl solution, H₂O and brine, dried over Na₂SO₄ and concentrated. The residue was chromatographed on silica gel (*n*-hexane-EtOAc 5:1) to afford **30** (5.81 g, 92%) as a white solid: mp 100-101 °C; ¹H NMR (CDCl₃) δ 0.08 (2s, 12H), 0.89 (s, 9H), 0.90 (s, 9H), 1.43 (ddd, 1H, *J*= 13.9, 10.0, 2.8 Hz), 1.62 (tt, 1H, *J* = 10.2, 2.0 Hz), 1.71 (ddd, 1H, *J* = 12.8, 10.6, 2.2 Hz), 2.24 (br-d, 1H, *J* = 13.9 Hz), 2.69 (br-d, 1H, *J* = 12.8 Hz), 3.01 (s, 3H), 3.74 (dd, 1H, *J* = 9.9, 2.2 Hz), 3.89 (m, 1H), 3.91 (dd, 1H, *J* = 9.9, 1.8 Hz), 4.19 (td, 1H, *J* = 10.0, 4.7 Hz), 4.45 (d, 1H, *J* = 12.0 Hz), 4.57 (d, 1H, J= 12.0 Hz), 5.13 (td, 1H, *J* = 10.6, 4.8 Hz), 7.33 (m, 5H); ¹³C NMR (CDCl₃) **δ** -5.6 (q), -5.3 (q), -4.6 (q), -3.7 (q), 17.9 (s), 25.8 (q), 35.5 (t), 38.5 (t), 38.8 (q), 51.8 (d), 56.9 (t), 65.1 (d), 70.1 (t), 72.0 (d), 77.5 (d), 127.4 (d), 128.4 (d), 138.5 (s); LISMS(GLY/NBA) 559 (M+H)⁺; HRMS calcd for C₂₇H₅₁O₆SSi₂ (M+H)⁺ 559.2945, found 559.2979; Anal. Calcd for C₂₇H₅₁O₆SSi₂: C 58.02, H 9.02; Found: C 57.96, H 8.82.

### (1S,3R,4R,5S)-4-tert-Butyldimethylsilyloxymethyl-5-tert-butyldimethylsilyloxy-3-methanesulfonyloxy-cyclohexanol (31)

A mixture of **30** (3.5 g, 6.27 mmol), Pd/C (10 %, 4.4 g) and HCOONH₄ (2.2 g) in MeOH (100 mL) was refluxed and 2 x 1.1 g of HCOONH₄ were added every 3 h interval. The reaction was refluxed until all the starting material was consumed (total 14 h). After cooling to rt, the reaction mixture was filtered through Celite® and the residue was washed with CH₂Cl₂ (3x). The filtrate was concentrated to afford crude **31** (2,83 g, 97%) as a white solid, which was used as such for the next step: mp 135-137 °C; ¹H NMR (CDCl₃) δ 0.08, 0.09 (2s, 12H), 0.89 (s, 9H), 0.92 (s, 9H), 1.43 - 1.68 (m, 3H), 1.83 (ddd, 1H, *J* = 13.2, 10.6, 2.8 Hz), 2.07 (br-d, 1H, *J* = 13.2 Hz), 2.44 (br-d, 1H, *J* = 13.2 Hz), 3.02 (s, 3H), 3.72 (dd, 1H, *J* = 10.0, 2.4 Hz), 3.90 (dd, 1H, *J* = 10.0, 2.4 Hz), 4.19 (td, 1H, *J* = 10.6, 4.1 Hz), 4.26 (m, 1H), 5.14 (td, 1H, *J* = 10.6, 4.7 Hz); ¹³C NMR (CDCl₃) δ -5.6 (q), -5.3 (q) , -4.7 (q), -3.8 (q), 17.9 (s), 25.8 (q), 38.8 (q), 38.9 (t), 40.8 (t), 51.7 (d), 57.1 (t), 64.9 (d), 65.5 (d), 77.3 (d); LISMS (GLY/NBA) 469 (M+H)⁺; HUMS calcd for C₂₀H₄₅O₆SSi₂ (M+H)⁺ 469.2475, found 469.2453; Anal. Calcd for C₂₀H₄₅O₆SSi₂: C 51.24, H 9.46; Found: C 51.24, H 9.36.

### (4R,5S)-4-tert-Butyldimethylsilyloxymethyl-5-tert-butyldimethylsilyloxy-cyclohex-2-en-1-one (33)

A mixture of crude **31** (2.83 g, 6.27 mmol) and MnO₂ (13.6 g, 156.8 mmol) in dry CH₂Cl₂ (100 mL) was stirred vigorously at rt for 21 h. The reaction mixture was filtered through Celite® and washed with CH₂Cl₂. The filtrate was concentrated and the residue was chromatoghraphed on silica gel (n-hexane-EtOAc 5:1, then 1:2) to yield starting material **30** (1.56 g, 53 %) and enone **33** (920 mg, 40% over two steps) as a light-yellow oil (solid upon storing in the refrigerator): ¹H NMR (CDCl₃) δ 0.07 (s, 12H), 0.89 (s, 18H), 2.50 (m, 1H), 2.46 (dd, 1H, *J* = 16.1, 10.6 Hz), 2.72 (dd, 1H, *J* = 16.1, 4.8 Hz), 3.73 (dd, 1H, *J* = 9.9, 5.6 Hz), 3.85 (dd, 1H, *J* = 9.9, 4.4 Hz), 4.09 (ddd, 1H, *J* = 10.6, 8.1, 4.8 Hz), 6.06 (dd, 1H, *J =* 10.2, 2.6 Hz), 6.88 (dd, 1H, *J* = 10.2, 2.6 Hz); ¹³C NMR (CDCl₃) δ -5.6 (q), -5.5 (q), -5.1 (q), -4.4 (q), 17.8 (s), 18.2 (s), 25.6 (q), 25.8 (q), 47.1 (t), 48.0 (d), 61.8 (t), 68.0 (d), 130.2 (d), 150.6 (d), 199.0 (s); LISMS (THGLY/NBA) 371 (M+H)⁺; HRMS calcd for C₁₉H₃₉O₃Si₂ (M+H)⁺ 371.2438, found 371.2432.

### (1R,4R,5S)-5-(tert-Butyldimethylsilyloxy)-4-(tert-butyldimethylsilyloxymethyl)-cyclohex-2-en-1-ol (34)

To a solution of **33** (920 mg, 2.49 mmol) in MeOH (35 mL) at rt under N₂ was added CeCl₃.7H₂O (1.39 g, 3.73 mmol). The mixture was stirred for 0.5 h and a clear solution was obtained. NaBH₄ (113 mg, 2.99 mmol) was added in portions and H₂ evolved. The reaction mixture was stirred for 1 h and quenched with H₂O. The mixture was stirred for 15 min and concentrated. The residue was diluted with EtOAc, washed with H₂O and brine, dried over Na₂SO₄ and concentrated. The residue was chromatographed on silica gel (n-hexane-EtOAc 10:1) to give **34** (844 mg, 91%) as a colourless oil: ¹H NMR (500 MHz, CDCl₃) δ 0.04 (s, 3H), 0.05 (s, 3H), 0.10 (s, 3H), 0.11 (s, 3H), 0.89 (s, 9H), 0.90 (s, 9H), 1.94 (ddd, 1H, *J* = 13.7, 5.3, 3.9 Hz), 1.99 (ddd, 1H, *J* = 13.7, 4.5, 2.6 Hz), 2.36 (m, 1H), 2.94 (d, 1H, *J* = 9.8 Hz), 3.38 (dd, 1H, *J* = 10.1, 7.8 Hz), 3.56 (dd, 1H, *J* = 10.1, 5.0 Hz), 4.09 (pseudo sext, 1H, *J* = 9.8, 4.5, 4.0, 3.9 Hz), 4.20 (pseudo pent, 1H, *J* = 5.3, 3.4, 2.6 Hz), 5.61 (dd, 1H, *J* = 10.0, 3.9 Hz), 5.95 (ddd, 1H, *J* = 10.0, 4.0, 1.8 Hz); ¹³C NMR (CDCl₃) δ - 5.5 (q), -5.4 (q), -4.9 (q), -4.8. (q), 18.0 (s), 18.3 (s), 25.8 (q), 25.9 (q), 35.6 (t), 46.5 (d), 63.5 (t), 64.8 (d), 67.7 (d), 127.0 (d), 131.1 (d); LISMS (THGLY/NBA) 373 (M+H)⁺; HRMS calcd for C₁₉H₄₀O₃Si₂ (M+H)⁺ 373.2594, found 373.2626; Anal. Calcd for C₁₉H₄₀O₃Si₂: C 61.23, H 10.82; Found: C 61.34, H 10.83.

### 9-[(1S,4R,5S)-5-(tert-Butyldimethylsilyloxy)-4-(tert-butyldimethylsilyloxymethyl)-2-cyclohexenyl]adenine (35a)

To a mixture of **34** (660 mg, 1.774 mmol), adenine (480 mg, 3.55 mmol) and PPh₃ (931 mg, 3.55 mmol) in dry dioxane (20 mL) under N₂ at rt was added a solution of DEAD (565 µL, 3.55 mmol) in dry dioxane (10 mL) over a period of 45 min. The reaction mixture was stirred at rt overnight, concentrated and the residue was chromatographed on silica gel (CH₂Cl₂-MeOH 50:1, then 20:1) to yield crude **35a** (960 mg) as a yellow foam: ¹H NMR (CDCl₃) δ -0.12 (s, 3H), -0.06 (s, 3H), 0.10 (s, 3H), 0.11 (s, 3H), 0.83 (s, 9H), 0.94 (s, 9H), 2.01 - 2.25 (m, 2H), 2.32 (m, 1H), 3.73 (dd, 1H, *J* = 9.9, 4.8 Hz), 3.82 (dd, 1H, *J* = 9.9, 4.4 Hz), 3.97 (ddd, 1H, *J =* 10.2, 7.0, 4.0 Hz), 5.37 (m, 1H), 5.73 (s, 2H), 5.88 (ddd, 1H, *J* = 9.9, 3.7, 2.5 Hz), 6.06 (ddd, 1H, *J* = 9.9, 2.2, 1.1 Hz), 7.86 (s, 1H), 8.39 (s, 1H); ¹³C NMR (CDCl₃) δ -5.5 (q), -5.4 (q), -5.0 (q), -4.6 (q), 17.8 (s), 18.3 (s), 25.6 (q), 25.9 (q), 36.5 (t), 47.2 (d), 49.6 (d), 62.9 (t), 64.5 (d), 120.2 (s), 124.4 (d), 134.9 (d), 139.9 (d), 149.8 (s), 153.0 (d), 155.5 (s); LISMS (THGLY/NBA) 490 (M+H)⁺; HRMS calcd for C₂₄H₄₄N₅O₂Si₂ (M+H)⁺ 490.3034, found 490.3058.

### 9-[(1S,4R,5S)-5-Hydroxy-4-hydroxymethyl-2-cyclohexenyl)adenine (36)

Crude **35a** was treated with TFA-H₂O (3:1, 40 mL) at rt overnight. The reaction mixture was concentrated and co-evaporated with toluene (2x). The residue was chromatographed on silica gel (CH₂Cl₂-MeOH 20:1, then 5:1) to afford **36** (149 mg, 54% over two steps):
Mp 90 - 92 °C; ¹H NMR (CD₃OD) δ 2.01-2.33 (m, 3H) , 3.80 (d, 2H, *J* = 4.8 Hz), 3.84 (m, 1H), 5.33 (m, 1H), 5.94 (ddd, 1H, *J* = 9.9, 3.7, 2.6 Hz), 6.13 (ddd, 1H, *J* = 9.9, 2.5, 1.4 Hz), 8.09 (s, 1H), 8.21 (s, 1H); ¹³C NMR (CD₃OD) δ 37.3 (t), 47.9 (d), 51.1 (d), 63.1 (t), 64.7 (d), 120.6 (s), 125.3 (d), 136.1 (d), 141.6 (d), 150.4 (s), 153.7 (d), 157.5 (s); UV λₘₐₓ (MeOH) = 260 nm; LISMS (THGLY/NBA) 262 (M+H)⁺; HRMS calcd for C₁₂H₁₆N₅O₂ (M+H)⁺ 262.1304, found 262.1359; Anal. Calcd for C₁₂H₁₆N₅O₂.0.7H₂O: C 52.62, H 6.04, N 25.57; Found: C 52.62, H5.95, N 25.77.

### 9-[(1R,3S,4R)-3-Hydroxy-4-hydroxymethylcyclohexanyl]adenine (2a)

A mixture of **36** (45 mg, 0.17 mmol) and Pd/C (10%, 40 mg) in MeOH (5 mL) was stirred under H₂ at rt for 24 h. The reaction mixture was cooled to rt and filtered through Celite® and washed with MeOH. The filtrate was concentrated and theireyidue was purified by reversed-phase HPLC (5% CH₃*CN* in *H*_{*2*}*O)* to yield **2a** (35 mg, 78%) as a white foam: ¹H NMR (CD₃OD) δ 1.71 (m, 1H), 1.87-2.18 (m, 5H), 2.39 (m, 1H), 3.69 (dd, 1H, *J* = 14.0, 7.3 Hz), 3.74 (dd, 1H, *J* = 14.0, 6.9 Hz), 4.12 (m, 1H), 4.87 (m, 1H, overlapped with HOD), 8.18 (s, 1H), 8.21 (s, 1H); ¹³C NMR (CD₃OD) δ 22.6 (t), 28.7 (t), 36.1 (t), 53.6 (d), 51.9 (d), 63.3 (t), 68.4 (d), 120.4 (s), 141.1 (d), 150.6 (s), 153.5 (d), 157.4 (s); LISMS (THGLY/NBA) 264 (M+H)⁺; HRMS calcd for C₁₂H₁₈N₅O₂ (M+H)⁺ 264.1460, found 264.1449.

Figure 1. Mechanism of Pd (0) coupling reaction which may yield the desired compound C.

Figure 2. ¹H NMR experiment demonstrates the solvent-dependent conformational equilibrium of compound **34.**

Figure 8 (a) TBDMSCl (1.2 eq), imidazole (2 eq), DMF, r.t., 48% starting from **6a**; (b) MsCl, Et₃N, CH₂Cl₂, 0 °C, 93 %; (c) DBU, toluene, or KOH, H₂O / THF; (d) DEAD, PPh₃, THF; (e) I₂, PPh₃, imidazole, toluene, reflux, 34%; (f) DBU, toluene, reflux, 68%.

Figure 9 (a) (CH₃)₂C(OCH₃)₂, PPTS, acetone/THF (1:2), r.t., 94%; (b) Pd-C (10%), HCOONH₄, MeOH, reflux, 100%; (c) PDC, CH₂Cl₂, r.t., 94%; (d) TBAF, THF, r.t.; (e) KF, 18-Crown-6, THF, r.t., 62% **19**; (f) CeCl₃.7H₂O, NaBH₄, MeOH, 90%; (g) PPh₃, DEAD, AcOH, THF; (h) Ac₂O, DMAP, CH₂Cl₂, 0 °C, 95%; (i) Adenine, NaH, Pd(PPh₃)₄, DMF/THF; (j) PPTS, MeOH, r.t., 59%; (k) Bz₂O, DMAP, CH₂Cl₂, 0 °C, 95%.

Figure 10 (a) TBDMSCl (1.2 eq), imidazole (1.5 eq), DMF, r.t., 70%; (b) MsCl, Et₃N, CH₂Cl₂, 0 °C, 100%; (c) Pd-C(10%), HCOONH₄, MeOH, reflux, 76%; (d) MnO₂, CH₂Cl₂, r.t., 48% and 47% recovery of **31**: (e) NaBH₄, CeCl₃.7H₂O, MeOH, 0 °C → r.t., 100%.

### Sodium salt of Ethyl-β-hydroxyacrylate sodium salt (1)

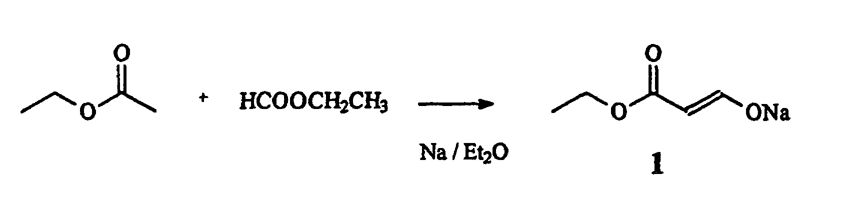

In a 1 L three necked flask, under inert atmosphere and equipped with an addition funnel, a well stirred suspension of fresh sodium pieces (23.0 g, 1.0 mol) in dry diethyl ether (400 mL) was prepared. A mixture of ethyl acetate (88.0 g, 1.0 mol) and ethyl formate (74.0 g, 1.0 mol) was added dropwise over a period of 45 minutes. Stirring was continued for an additional 14 hours using an ice bath, avoiding the reaction to become too vigorous. The resulting suspension was kept in the refrigerator for 8 hours, after which it was filtered, washed with dry diethyl ether (100 mL) and dried *in vacuo* to obtain 1 as a pale yellow solid (85.0 g, 61% yield).

### Ethyl β-acetoxyacrylate [cis (2') + trans (2)]

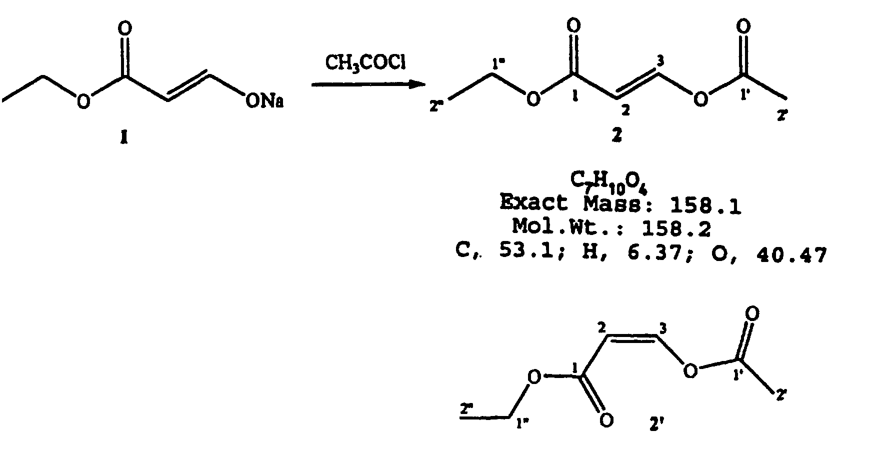

In a 2 L flask on an ice-bath, under an inert atmosphere, a well stirred suspension of the sodium salt **1** (85.0 g, 616 mmol) was prepared in dry diethyl ether (850 mL), to which acetyl chloride (52.9 mL, 58.2 g, 739 mmol) was added dropwise over 15 minutes. The mixture was stirred for an additional 6 hours, after which it was neutralized with a saturated aqueous solution of NaHCO₃ (250 mL). Both phases were separated and the aqueous phase was extracted with diethyl ether (5 x 200 mL). The combined organic phases were dried over Na₂SO₄, filtered and evaporated *in vacuo* to obtain a residual red oil (59.1 g). Distillation *in vacuo* (70 °C, 1 Torr aprox.) afforded a mixture of **2** and **2**', as a pure colorless oil (36.5 g, 23 % yield in two steps) with a *cis l trans* proportion of 4:10 (¹H-NMR).
analytical data of 2 (*trans* isomer)
¹H-NMR (200 MHz, CDCl₃) δ: 1.30 (t, *J* = 7.2 Hz, 3H, 2"-H), 2.22 (s, 3H, 2'-H), 4.21 (q, *J* = 7.2 Hz, 2H, 1"-H), 5.72 (d, *J* = 12.6 Hz, 1H, 2-H), 8.30 (d, *J* = 12.6 Hz, 1 H, 3-H).
analytical data of 2' (*cis* isomer)
¹H-NMR (200 MHz, CDCl₃) δ: 1.30 (t, *J* = 7.2 Hz, 3H, 2"-H), 2.28 (s, 3H, 2'-H), 4.20 (q, *J* = 7.4 Hz, 2H, 1"-H), 5.30 (d, *J* = 7.3 Hz, 1H, 2-H), 7.54 (d, *J* = 7.3 Hz, 1H, 3-H).

### Isomerization of the cis/trans mixture (2/2') to ethyl trans-β-acetoxyacrylate (2)

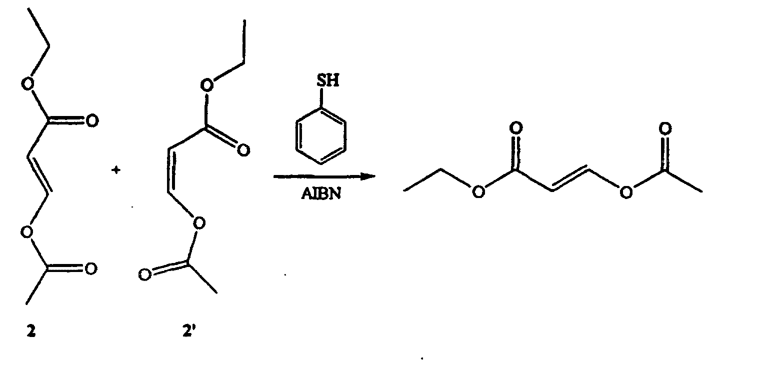

In a well-closed flask, under magnetic stirring, the **2/2'** mixture obtained from several operations (52.5 g, 39:100 *cis* / *trans* proportion, 332 mmol) was treated with thiophenol (16.3 ml, 17.5 g, 159 mmol) and 2,2'-azobis(2-methylpropionitrile) (AIBN, 8.31 g, 50.6 mmol) and the mixture was heated to 80 °C for 2.5 hours. The flask was cooled for 2 hours and the crude was diluted with ethyl acetate (400 mL) and washed with an aqueous solution of NaOH 0.01 N (400 mL). The organics were dried over Na₂SO₄, filtered and evaporated *in vacuo* to leave a pale yellow oil. Distillation *in vacuo* (53 °C, 0.5-1.0 Torr) afforded 2 (55.8 g, quantitative yield) with a cis / trans proportion of 3:97 (¹H-NMR), slightly contaminated with aromatic sulphurated products.

### Preparation of (E)-1-methoxy-3-trimethylsilyloxy-1,3-butadiene (4)

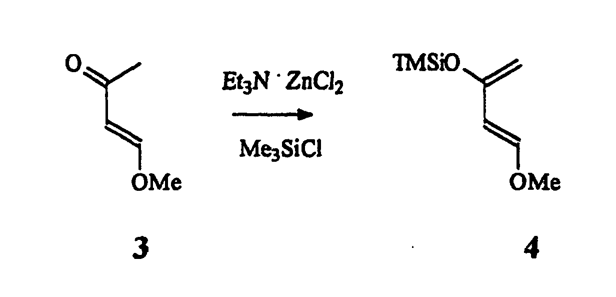

Under an inert atmosphere anhydrous ZnCl₂ (2.52 g, 18.5 mmol) was slowly added under magnetic stirring to triethylamine (distilled over KOH) (145 g, 200 mL, 1.43 mol). The mixture was stirred for 1 hour at room temperature until a fine suspension was obtained. A solution of compound **3** (63.1 g, 630 mmol) in toluene (190 mL) was then added over 5 min, followed by gradual addition of chlorotrimethylsilane (137.0 g, 160 mL, 1.26 mol) over a period of 10 min. An exothermic reaction was noted. After 30 minutes, the temperature was raised to 40 °C and stirring was continued overnight. Following cooling, the reaction mixture was diluted with diethyl ether (1 L), filtered and washed with diethyl ether (4 x 100 mL). The combined filtrate and ether washings were concentrated *in vacuo* to leave a brown oil. Distillation trough a Vigreux column (52 °C, 1.0 Torr) afforded compound **4** in a middle cut, slightly contaminated with compound **3** [80.1 g, 91% purity (¹H-NMR), 67% yield of 4].
NOTE: compound **4** is commercially available (e.g. Aldrich®).
analytical data of **4**.
¹H-NMR (200 MHz, CDCl₃) δ: 0.23 [s, 9H, OSi(CH₃)₃], 3.59 (s, 3H, OCH₃), 4.09 (d, *J =* 8.2 Hz, 2H, 4-H), 5.35 (d, *J =* 12.2 Hz, 1H, 2-H), 6.83 (d, *J =* 12.2 Hz, 1H, 3-H).

### Diels-Alder adduct of 2 and 4:

### 5-O-acetyl-4-ethoxycarbonyl-3-O-methyl-1-O-trimethylsilyl-cyclohexen-1,3,5-triol

### [(±) 5a + 5b]

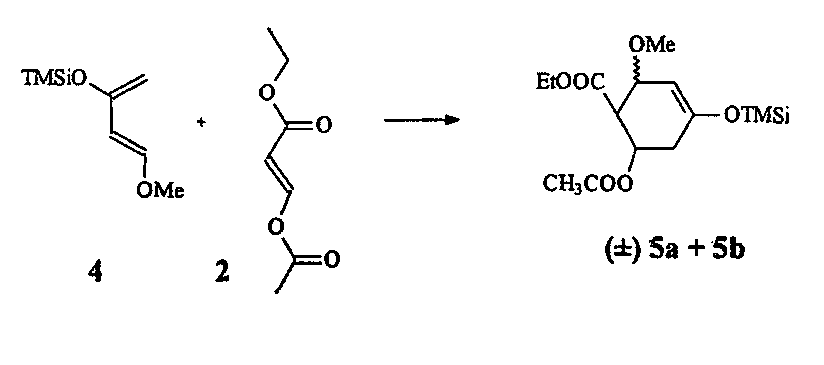

In a 250 mL round bottom flask a small amount of hydroquinone (372 mg) was added under magnetic stirring to a mixture of the Danishefky diene [**4**, 72.9 g, 91% purity (¹H-NMR), 385 mmol] and **2** (55.8 g, *cis*/*trans* 3:97, 353 mmol) and the mixture was heated at 180 °C for 1.5 hours. An additional amount of 372 mg of hydroquinone was added and the reaction mixture was distilled *in vacuo* (94 °C, 3.0 x 10⁻² mm Hg) to afford a slightly contaminated mixture of (±) **5a** + **5b** (72.0 g, 62% yield), with the substituents at the 4- and 5-position oriented in *trans*.

NOTE: Upon increasing the temperature of the distillation bath to 170 °C or higher, different quantities of the phenolic derivative **6** are obtained. The phenol derivative 6 likewise is obtained as the main isolated product when purification on silica gel is undertaken. The addition of fresh hydroquinone right before the distillation seems to avoid the formation of **6**. Compound **6** could not detected by NMR when using this improved procedure.

### representative analytical data for the major derivative (±) 5a (substituents at 3 and 4 in trans)

¹H NMR (CDCl₃) δ 0.21 (s, 9H), 1.27 (t, 3H, *J* = 7.3 Hz), 2.01 (s, 3H), 2.19 (m, 1H), 2.55 (dd, 1H, *J =* 16.7, 5.5 Hz), 2.77 (dd, 1H, *J =* 11.4, 8.4 Hz), 3.31 (s, 3H), 4.20 (m, 2H), 4.35 (dm, 1H, *J* = 8.4 Hz), 4.94 (t, 1H, *J* = 2.2 Hz), 5.13 (ddd, 1H, *J* = 11.0, 9.2, 5.9 Hz).
¹³C NMR (CDCl₃) δ 0.06 (q), 14.2(q), 20.8 (q), 35.4 (t), 51.1 (t), 55.4 (q), 60.9 (t), 68.8 (d), 76.5 (d), 103.3 (d), 149.3 (s), 170.0 (s), 172.2 (s).

### Ethyl p-hydroxybenzoate (6) : analytical data

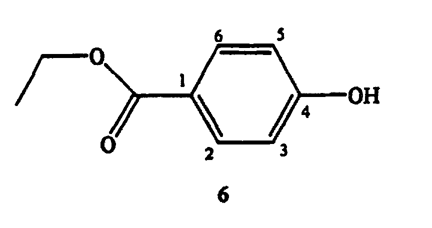

¹H-NMR (200 MHz, CDCl₃) δ: 1.39 (t, *J* = 7.2 Hz, 3H, -CH₂CH₃), 4.37 (q, 2H, -CH₂CH₃), 6.91 [d, *J* = 8.9 Hz, 2H, 3(5)-H], 7.36 (broad s, 1H, 4-OH), 7.96 [d, *J* = 8.9 Hz, 2H, 2(6)-H].
¹³C-NMR (50.3 MHz, CDCl₃) δ: 14.1 (-CH₂CH₃), 61.1 (-CH₂CH₃), 115.3 [C3(5)], 122.2 (C1), 132.0 [C2(6)], 160.7 (C4), 167.6 (C=O).

### (±) 4-hydroxymethyl-cyclohex-2-en-1,5-diol (7a)

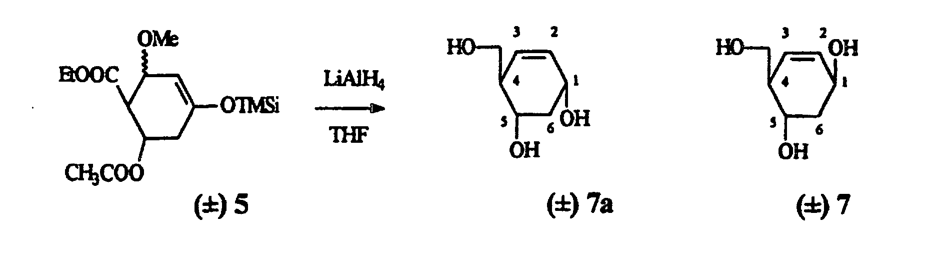

In a 1L three necked bottom flask on a ice-NaCl bath, a suspension of LiAlH₄ (25.0 g, 658 mmol) in dry THF (220 mL) was prepared under magnetic stirring in an inert atmosphere. To this cooled suspension, a solution of the impure mixture of **5a** + **5b** (27.2 g) in dry THF (85 mL) was added dropwise during 30 minutes. After stirring at 0 °C for 2 hours, the reaction was continued at room temperature for an additional 19 hours. The mixture became very viscous and was diluted with dry THF (110 mL). After cooling on an ice-NaCl bath, the mixture was treated consecutively and very carefully (equipping the system with a good gas-exit) with water (25 mL), stirring for 15 minutes, with 15% aqueous NaOH (25 ml), stirring for 15 minutes more, and finally with more water (75 ml). A dry granular precipitate was produced, which was easy to filter and wash. The suspension was stirred for 30 minutes and the precipitate was filtered over a layer of Celite®, and washed with water (5 x 100 mL) and ethyl acetate (3 x 100 mL). Both phases were separated and the aqueous phase was washed with ethyl acetate (3 x 100 mL). The aqueous phase was evaporated to dryness to give a brown gummy residue (21.1 g) which was filtered through a silica gel column (210 g) packed with ethyl acetate, eluting with mixtures of EtOAc/MeOH of increasing polarity. The title product **7a** was isolated as a pale yellow oil (3.55g, 24.7 mmol, 30%), preceded by its epimer **7b** (6.44g) as an impure mixture.
analytical data of 7a
¹H NMR (CDCl₃ + DMSO-*d*_{*6*}) δ 1.48 (td, 1H, *J* = 11.3, 9.2 Hz), 2.02 - 2.23 (m, 2H), 3.35 (m, 1H), 3.61 (m, 2H), 3.75 (d, 1H, *J* = 5.8 Hz, OH), 4.01 (t, 1H, *J* = 4.6 Hz, OH), 4.11 (m, 1H), 4.20 (d, 1H, *J* = 3.3 Hz, OH), 5.25 (dt, 1H, J = 9.9, 2.0 Hz), 5.58 (dm, 1H, *J* = 9.9 Hz).
¹³C NMR (CDCl₃ + DMSO-*d*_{*6*}) δ 39.7 (t), 45.8 (d), 65.2 (t), 65.9 (d), 69.4 (d), 126.1 (d),
132.4 (d).
LISMS (THGLY/NaOAc) 167 (M+Na)⁺ (C₇H₁₂O₃)
Data of **7b:**
¹H NMR (DMSO-*d*₆) δ 1.37 (td, 1H, *J* = 11.7, 9.9 Hz), 1.92 - 2.10 (m, 2H), 3. 24 - 3.45 (m, 2H), 3.63 (dt, 1H, J = 10.2, 4.4 Hz), 4.07 (m, 1H), 4.49 (t, 1H, *J* = 5.3 Hz, OH), 4.63 (d, 1H, *J* = 5.1 Hz, OH), 4.70 (d, 1H, *J* = 5.9 Hz, OH), 5.52 (d, 1H, *J* = 11.0 Hz), 5.57 (d, 1H, *J* = 11.0 Hz).
¹³C NMR (DMSO-ds) δ 42.0 (t), 47.2 (d), 62.2 (t), 65.9 (d), 66.3 (d), 127.7 (d), 132.8 (d).
LISMS (THGLY/TFA) 145 (M+H)⁺ (C₇H₁₂O₃)

### Formation of the ketal of 7a:

### (±) 5,7-O-benzylidene-4-hydroxymethyl-cyclohex-2-en-1,5-diol (8)

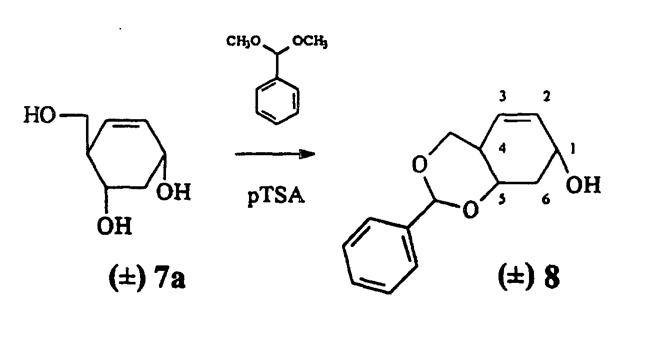

Under an inert atmosphere benzaldehyde dimethyl acetal (6.2 mL, 41.2 mmol) and *p*-toluenesulfonic acid monohydrate (300 mg, 1.58 mmol) were added to a solution of (±)**7a** (4.49 g, 31.1 mmol) in dry dioxane (140 mL). The mixture was stirred at room temperature for 24 hours and subsequently poured into ethyl acetate (100 mL), washed with water (250 mL), dried over Na₂SO₄ and concentrated to give a white residue (8.91 g). Chromatographic purification on silica gel (270 g) eluting with mixtures of hexane/EtOAc of increasing polarity afforded the desired product **8** as a white crystalline solid (5.06 g, 70% yield, 80% yield based on recovered **7a**).
The aqueous phase was evaporated to dryness, to recover the starting material **7a** (600 mg, 13 % recovery).
analytical data of 8a
¹H-NMR (500 MHz, CDCl₃) δ: 1.60 (d, *J* = 7.3 Hz, 1H, OH), 1.80 (ddd, 1H, 6-H_{*a*}), 2.53 (ddd, 1H, 6-H_{*e*}), 2.60 (m, 1H, 4-H), 3.61 (t, *J* = 11.2 Hz, 1H, 7-H_{*a*}), 3.68 (ddd, 1H, 5-H), 4.26 (dd, *J* = 10.7 and 4.4 Hz, 1H, 7-H_{*e*}), 4.53 (m, 1H, H-1), 5.42 (ddd, *J* = 9.7 Hz, 1H, 2-H), 5.59 (s, 1H, PhCH), 5.74 (ddd, *J* = 9.8 Hz, 1H, 3-H), 7.31-7.40 and 7.48-7.53 (m, 5H, arom-H).
¹³C-NMR (500 MHz, CDCl₃) δ: 38.6 (C-6), 40.1 (C-4), 68.0 (C-1), 70.7 (C-7), 77.7 (C-5), 102.2 (PhCH), 125.0 (C-2), 126.2 (ar-Cₒ), 128.3 (ar-Cₘ), 129.0 (ar-Cᵢ), 132.7 (C-3), 138.1 (ar-Cₚ).
LISMS (GLY/TFA) 233 (M+H)⁺ (C₁₄H₁₆O₃)

Additional amounts of the desired 8a can be obtained using the other epimer **7b**, using an oxidation - reduction cycle as outlined below.

Therefore, the crude **7b** (2.3 g, 14.58 mmol) was treated with benzaldehyde dimethyl acetal (3.28 mL, 21.87 mmol) in the presence of p-toluenesulfonic acid monohydrate (PTSA, 138 mg, 0.73 mmol) in 1,4-dioxane (30 mL) at r.t. for two days. Ice was added and the mixture was stirred at r.t. for 0.5 hr and extracted with EtOAc (3x ). The combined organic solvents were washed with water and brine, dried over sodium sulfate and concentrated. The residue was purified on silica gel (hexane /EtOAc 1:1) to afford a mixture of **8b** and **8a** (3:1, 1.2 g) as a light yellow solid.

The mixture of **8a/8b** (3:1, 415 mg, 1.79 mmol) and MnO₂ (1.56 g, 17.9 mmol, 10 eq) in dry CH₂Cl₂ (15 mL) was stirred at rt for 21 hrs. The reaction mixture was diluted with CH₂Cl₂ and filtered through Celite. The filtrate was concentrated and the residue was chromatographed on silica gel (hexane - EtOAc 2:1) to afford **9** (340 mg, 83%) as a colourless oil.
¹H NMR (CDCl₃) δ 2.65 (dd, 1H, *J* = 16.4, 13.1 Hz), 2.83 (m, 1H), 2.95 (dd, 1H, *J* = 16.4, 4.8 Hz), 3.79 (t, 1H, *J* = 11.1 Hz), 4.04 (ddd, 1H, *J* = 13.1, 9.2, 4.8 Hz), 4.45 (dd, 1H, J = 11.1, 4.8 Hz), 5.63 (s, 1H), 6.13 (dd, 1H, *J* = 9.9, 2.9 Hz), 6.58 (dd, 1H, *J* = 9.9, 1.8 Hz), 7.39 (m, 3H), 7.51 (m, 2H).
¹³C NMR (CDCl₃) δ 39.9 (d), 44.3 (t), 69.2 (t) , 77.4 (d), 101.7 (d), 126.1 (d), 128.4 (d), 129.2 (d), 132.1 (d), 137.5 (s), 144.9 (d), 196.8 (s).
LISMS (NBA) 231 (M+H)⁺. (C₁₄H₁₄O₃)

### Conversion of 9 to 8a:

To a solution of **9** (340 mg, 1.5 mmol) in MeOH (15 mL) at rt was addded CeCl₃.7H₂O (838 mg, 2.25 mmol, 1.5 eq). After stirring at rt for 1 hr, NaBH₄ (68 mg, 1.8 mmol, 1.2 eq) was added in portions. The reaction was stirred at rt for 2 hrs and quenched with crushed ice. The resulting mixture was stirred at rt for 0.5 hr and concentrated. The residue was taken into ethyl acetate and washed with water and brine, dried over sodium phosphate and concentrated. The residue was chromatographed on silica gel (hexane - EtOAc 5:1 and 1:1) to give **8a** as a white solid which proved identical to the previous material.

The product **8a**, or its analogues, either under their racemic form, or under the form of their separated isomers, as represented by the general structure **III**, can be used for synthesis of cyclohexenyl nucleoside analogues of general structure **IV**, according to standard procedures for alkylation of heterocyclic bases. Hereto, in the general structure **III**, R¹ and R² are representing protecting groups (e.g. R₁, R₂ = C₆H₅-CH=), and R³ represents a leaving functionality (e.g. R³ = SO₂CH₃, SO₂CF₃, SO₂C₆H₄CH₃, SO₂C₆H₄CH₃, SO₂C₆H₄Br) enabling nucleophilic substitution reactions, or R³ represents hydrogen, to be used in Mitsunobu reactions.

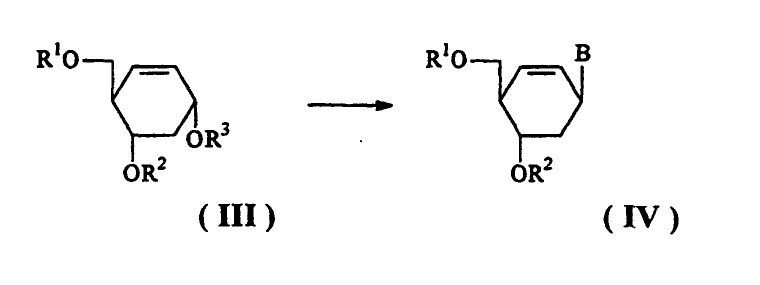

### example:

### N²-Benzoyl-9-(5-hydroxy-4-hydroxymethyl-2-cyclohexenyl)guanine ( (±) 11)

To a mixture of (±) **8a** (696 mg, 3 mmol), 2-amino-6-chloropurine (1.02 g, 6 mmol) and triphenyl phosphine (PPh₃, 1.57 g, 6 mmol) in dry 1,4-dioxane (30 mL) was added slowly a solution of DEAD (945 mL, 6 mmol) in dry 1,4-dioxane (10 mL). The reaction was stirred at r.t. overnight and concentrated. The residue was taken on silica gel and chromatographed on silica gel (CH₂Cl₂ / MeOH 100:1 and 50:1) to afford the crude **10** (2 g) and the N₇-epimer (140 mg) as a white solid.

The crude **10** (2 g) was treated with TFA/H₂O (3:1, 20 mL) at r.t. for 2 days. The reaction mixture was concentrated and coevaporated with toluene. The residue was chromatographed on silica gel (CH₂Cl₂ / MeOH 50:1 and 10:1) to produce (±) **11** (220 mg, 27 % overall yield starting from **8a**).

The spectrum of **11** is identical to that previously reported.

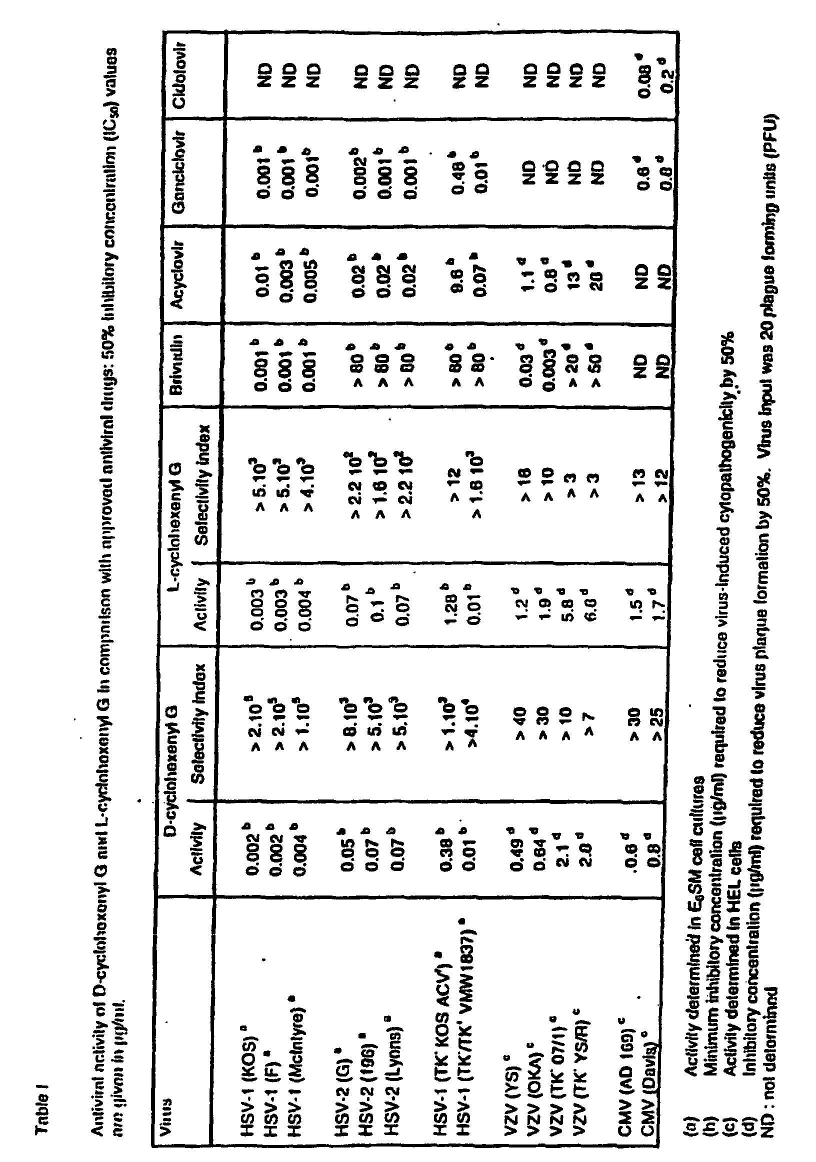

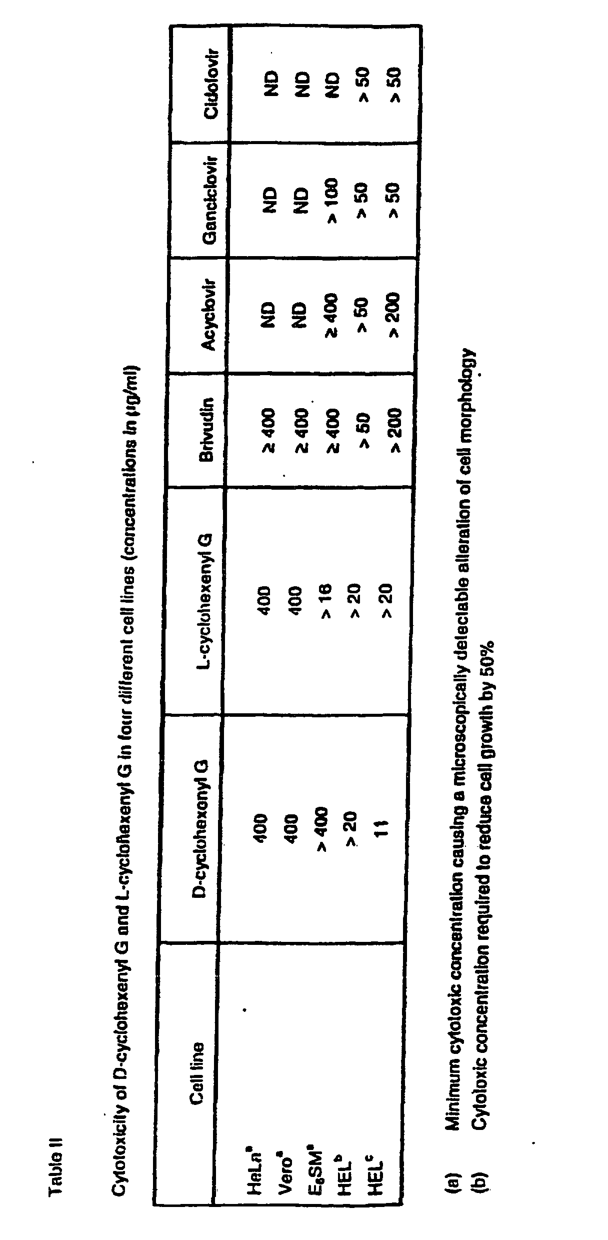

## Claims

1. A six membered, at least partially unsaturated, carbocyclic nucleoside compound, including the (-) enantiomer, the (+) enantiomer, and pharmaceutically acceptable salts and esters thereof, the compounds represented by the general formula I: 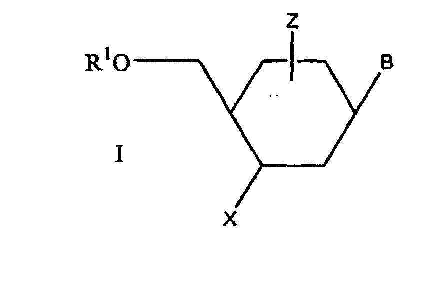 wherein:
- Z represents the presence of 1 or more double bonds in the six membered carbocyclic ring,
- B is a heterocyclic ring selected from the group consisting of pyrimidine and purine bases,
- X is a hydrogen, azido, F, or OR²,
- R¹ and R² are the same or different and represent the same or different protecting groups, hydrogen, alkyl, alkenyl, acyl or phosphate moieties
wherein;
- the alkyl moiety is a saturated, substituted or unsubstituted straight or branched chains hydrocarbon radical having from 1 to 20, for example 1-16, 1-14, 1-12, 1-10, 1-8, 1-4, carbon atoms,
- the alkenyl moiety is an unsaturated congener of the alkyl group and,
- the acyl moiety is an alkanoyl or aroyl moiety, wherein alkanoyl is an alkyl carbonyl radical, wherein alkyl is as described above and aroyl represents benzoyl substituted benzoyl or naphtoyl.

2. A six membered, at least partially unsaturated, carbocyclic nucleoside compound, according to claim 1, being a cyclhexenyl nucleoside compound having the general formula II or III:

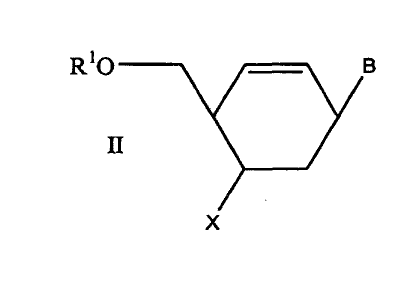

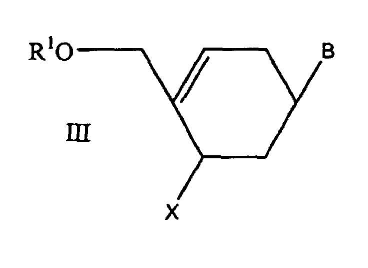

3. Compound according to claims 1 or 2, selected from the following group of compounds represented by the formulas IV-X':

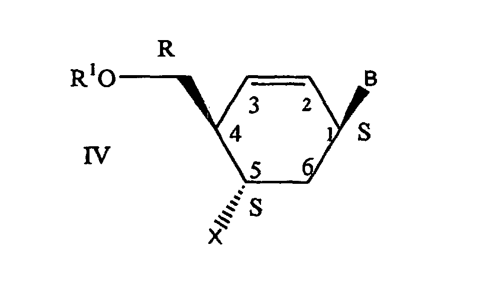

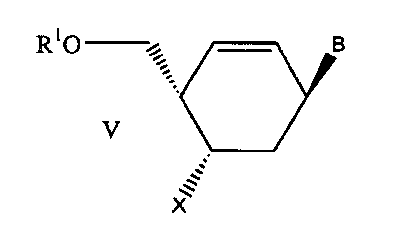

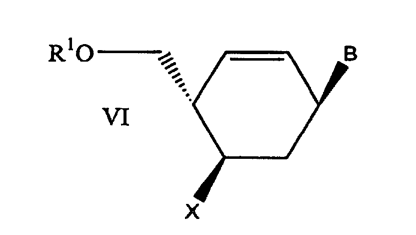

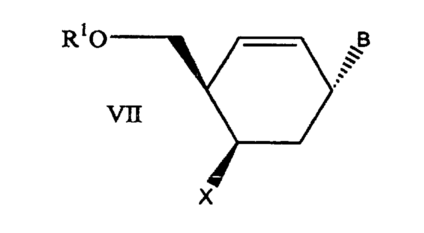

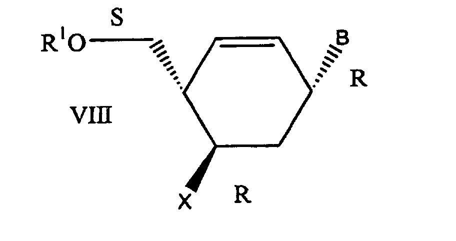

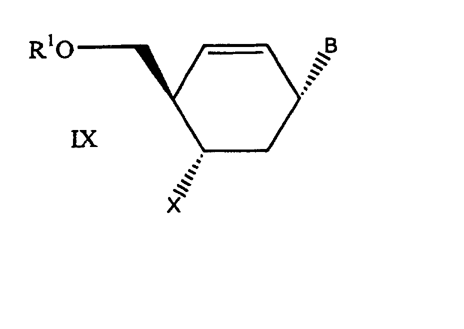

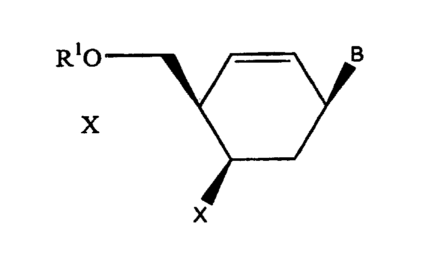

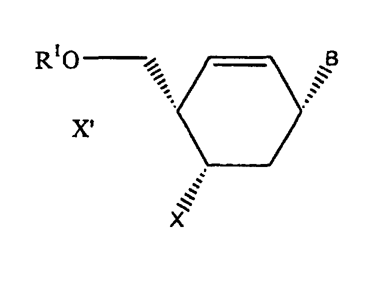

4. Compound according to any of the preceding claims, wherein the C₁ bearing B substituent and the C₅ bearing X substituent both have the (S)-configuration, and the C₄ bearing -OR¹ substituent has the (R)-configuration, as depicted by formula IV in claim 3.

5. Compound according to claims 1,2 or 3, wherein the C₁ bearing B substituent and the C₅ bearing X substituent both have the (R)-configuration, and the C₄ bearing -OR¹ substituent has the (S)-configuration, as depicted by formula VIII in claim 3.

6. Compound according to any of the claims 1-4, wherein X is represented by a hydroxyl group in the (S)-configuration.

7. Compound according to any of the claims 1, 2,3 and 5, wherein X is hydroxyl in the (R)-configuration.

8. Compound according to any of the preceding claims wherein B is derived from the group consisting of pyrimidine bases.

9. Compound according to claim 7 wherein the pyrimidine base has the general formula XI: 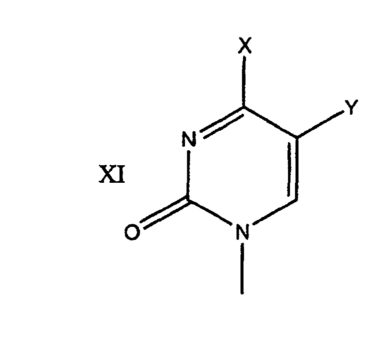 wherein X is chosen from the following;
- OH, NH₂, NHQ,
wherein;
- Q is selected from the following;
OH or C₁₋₅ alkyl,
- Y is selected from the following;
H, F, Cl, Br, I, C₁₋₅ alkyl, haloethyl or CH=CH-R, wherein R represents hydrogen, halogen or C₁₋₅ alkyl, and wherein haloethyl contains from 1 to 4 F, CI or Br atoms.

10. Compound according to any of the preceding claims wherein B is selected form the group consisting of substituted and unsubstituted adenine, guanine, 2,6-diaminopurine, hypoxanthine and xanthine.

11. Compound according to any of the preceding claims wherein the B is selected from the group of aza, deaza deoxy or deamino analogues of the heterocyclic rings, as defined in any of the claims 8-10.

12. Compound according to any of the preceding claims wherein the protecting group comprises a silyl protecting group, preferably TBDMS, and/or a benzoyl protecting group and or a C6H5-CH= group.

13. Compound according to any of the preceding claims 1-11 selected from:
- 9-[1S,4R,5S)-5-hydroxy-4-hydroxymethyl-2-cyclohexenyl] guanine
- 9-[1R,4S,5R)-5-hydroxy-4-hydroxymethyl-2-cyclohexenyl].

14. Compound according to any of the claims 1-11 selected from the following group:
- 9-[(1S,4R,5S)-5-(tert-Butyldimethylsilyloxy)-4-(tert-butyldimethylsilyloxymethyl)-2-cyclohexenyl]adenine
- 9-[(1S,4R,5S)-5-Hydroxy-4-hydroxymethyl-2-cyclohexenyl]adenine
- 9-[(1S,4R,5S)-5-(tert-butyldimethylsilyloxy)-4-(tert-butyldimethylsilyloxymethyl)-2-cyclohexenyl]-2-amino-6-chloropurine
- 9-[(1S,4R,5S)-5-hydroxy-4-hydroxymethyl-2-cyclohexenyl]guanine
- 9-[(1R,4S,5R)-5-Benzoyloxy-4-benzoyloxymethyl-2-cyclohexenyl]adenine
- 9-[(1R,4S,5R)-5-hydroxy-4-hydroxymethyl-2-cyclohexenyl]adenine
- 9-[(1R,4S,5R)-5-Benzoyloxy-4-benzoyloxymethyl-2-cyclohexenyl]guanine
- 9-[(1R,4S,5R)-5-Hydroxy-4-hydroxymethyl-2-cyclohexenyl]guanine

15. Process for providing a compound, the (-) enantiomer, the (+) enantiomer, and pharmaceutically acceptable salts and esters thereof according to any of the preceding claims, said process comprising the steps of:
- providing cyclohexenyl compound of the general formula XII; 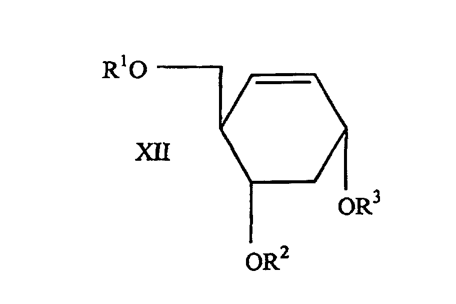
- wherein R¹ and R² are protecting groups and R³ is a leaving group or an Hydrogen atom, followed by the step of substituting the OR-³ group by a pyrimidine or purine base.

16. Process according to claim 15 wherein R³ is hydrogen and wherein a Mitsunobo type reaction is utilised.

17. Process according to claim 15 wherein R³ is a leaving group enabling nucleophilic substitution.

18. Process according to any of the preceding claims 15-17 wherein the compound of general formula XII has the chemical formula XIII; 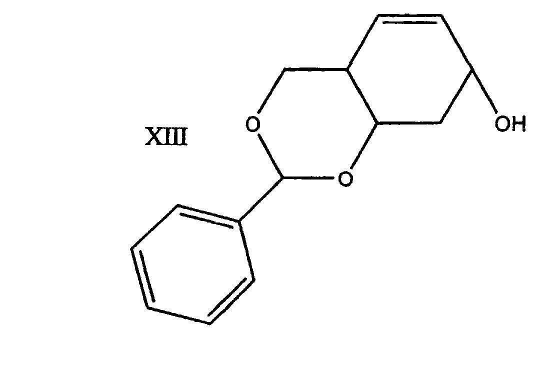 or analogues thereof either in a racemate form or separated isomers thereof.

19. Process according to any of the preceding claims 15-18 wherein compound XIII is provided by reacting (±) 4-hydroxymethyl- cyclohex-2-en-1,5 Diol of formula XIV; 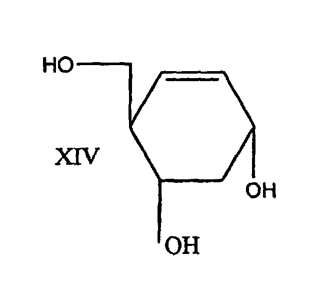 with a benzaldehyde analogue and a lewis acid, preferably being benzaldehyde dialkyl acetal, most preferably dimethyl acetal, and p-toluenesulfonic acid.

20. Process according to any of the preceding claims 15-19 wherein compound XIV is provided by the reduction of compound XVA or XVB, preferably utilising lithium aluminium hydride or an equivalent thereof as reducing agent; 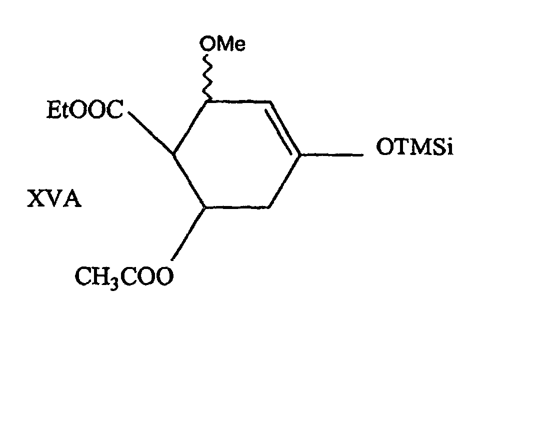 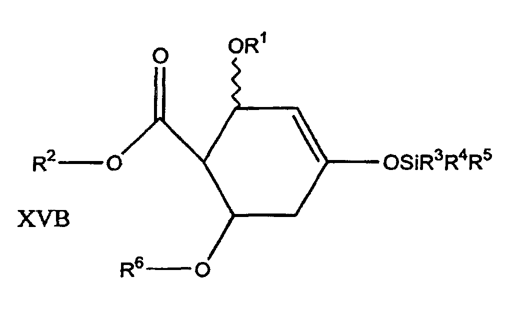 wherein for XVB:
R¹ and R² are alkyl or alkenyl moieties,
wherein:
- R¹ and R² are the same or different, and
- alkyl is a saturated, substituted or unsubstituted hydrocarbon radical having from 1 to 20 for example 1-16, 1-14, 1-12, 1-10, 1-8, 1-4, carbon atoms and being straight or branched chain, and
- alkenyl is the unsaturated congener of the alkyl group, and
R³, R⁴ and R⁵ are alkyl, alkenyl or aryl moieties, wherein:
- R³, R⁴ and R⁵ are the same or different, and
- alkyl is a saturated, substituted or unsubstituted straight or branched chain hydrocarbon radical having from 1 to 20 for example 1-16, 1-14, 1-12, 1-10, 1-8, 1-4, carbon atoms and
- alkenyl is the unsaturated congener of the alkyl group, and
- aryl represents phenyl or substituted phenyl, and
R⁶ is a alkyl, alkenyl or acyl moiety, wherein:
- alkyl is a saturated, substituted or unsubstituted hydrocarbon straight or branched chain radical having from having from 1 to 20 for example 1-16, 1-14, 1-12, 1-10, 1-8, 1-4, carbon atoms,
- alkenyl is the unsaturated congener of the alkyl group, and
- acyl is an alkanoyl or aroyl moiety, wherein alkanoyl is an alkyl carbonyl radical, wherein alkyl is as described above and aroyl represents benzoyl, substituted benzoyl or naphtoyl.

21. Process according to claim 20 wherein compound XVA or XVB is provided by a diels-alder reaction, by the cyclo addition of a suitable diene and dienophile wherein preferably the diene and dienophile are heated together in the presence of hydroquinone.

22. Process according to claim 21 wherein the diene has the following chemical structure XVI, and the dienophile has the following chemical structure XVII, wherein R¹, R², R³, R⁴, R⁵ and R⁶ are as defined in claim 20;

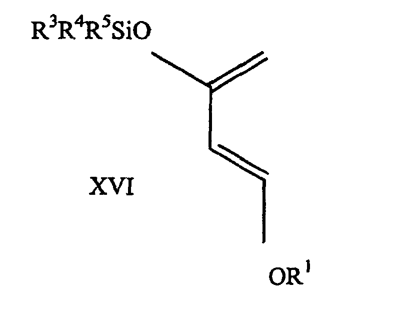

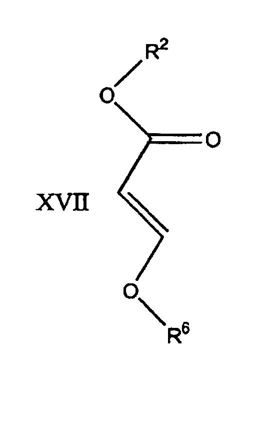

23. Process according to claim 22 wherein the diene has the chemical structure XVI' and the dienophile has the chemical structure XVIII;

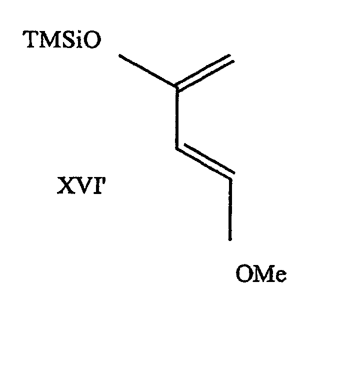

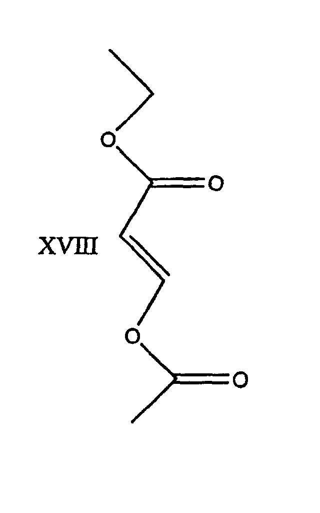

24. A six membered, at least partially unsaturated, carbocyclic nucleoside compound, including the (-) enantiomer, the (+) enantiomer, and pharmaceutically acceptable salts and esters thereof, the compounds represented by the general formula XII or XIX; 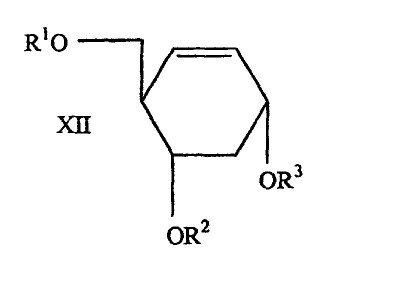 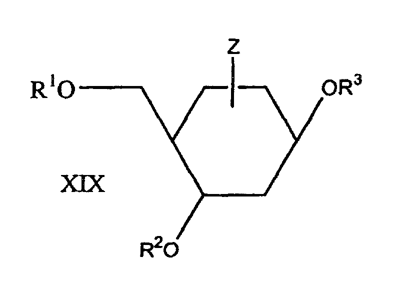 wherein:
- Z represents the presence of 1 or more double bonds in the carbocyclic ring,
- R¹ and R² are protecting groups and R³ is a leaving group or an Hydrogen atom.

25. Compound according to claim 24 wherein;
R¹ and R² are the same or different and hydrogen, alkyl, alkenyl, acyl or phosphate moieties are represented, or R¹ and R² represent a cyclic protecting group; wherein:
- alkyl is a saturated, substituted or unsubstituted straight or branched chain hydrocarbon radical having from 1 to 20 for example 1-16, 1-14, 1-12, 1-10, 1-8, 1-4, carbon atoms, and
- alkenyl is the unsaturated congener of the alkyl group, and
- acyl is an alkanoyl or aroyl moiety, wherein alkanoyl is an alkyl carbonyl radical, wherein alkyl is as described above and aroyl represents benzoyl, substituted benzoyl or naphtoyl; and
R³ represents a hydrogen, an alkylsulfonyl or an arylsulfonyl moiety, wherein:
- alkyl is a saturated, substituted or unsubstituted hydrocarbon radical having from 1 to 6 carbon atoms and straight or branched chain, and
- aryl represents phenyl or substituted phenyl, and

26. A cyclohexenyl compound, including the (-) enantiomer, the (+) enantiomer, and pharmaceutically acceptable salts and esters thereof, the compound represented by the general formula XVB; 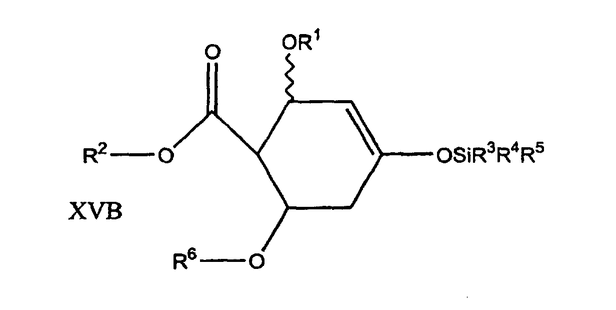 wherein R¹ and R² are alkyl or alkenyl moieties, wherein:
- R¹ and R² are the same or different, and
- alkyl is a saturated, substituted or unsubstituted straight or branched chain hydrocarbon radical having from 1 to 20 for example 1-16, 1-14, 1-12, 1-10, 1-8, 1-4 carbon atoms,
- alkenyl is the unsaturated congener of the alkyl group, and
R³, R⁴ and R⁵ are alkyl, alkenyl or aryl moieties, wherein:
- R³, R⁴ and R⁵ are the same or different, and
- alkyl is a saturated, substituted or unsubstituted straight or branched chain hydrocarbon radical having from 1 to 20 for example 1-16, 1-14, 1-12, 1-10, 1-8, 1-4 carbon atoms and,
- alkenyl is the unsaturated congener of the alkyl group, and
- aryl represents phenyl or substituted phenyl, and
R⁶ is a alkyl, alkenyl or acyl moiety, wherein:
- alkyl is a saturated, substituted or unsubstituted straight or branched chain hydrocarbon radical having from 1 to 20 for example 1-16, 1-14, 1-12, 1-10, 1-8, 1-4 carbon atoms, and
- alkenyl is the unsaturated congener of the alkyl group, and
- acyl is an alkanoyl or aroyl moiety, wherein alkanoyl is an alkyl carbonyl radical, wherein alkyl is as described above and aroyl represents benzoyl, substituted benzoyl or naphtoyl.

27. Compound according to claim 26 having the formula XVA being; 5-O-acetyl-4-ethoxycarbonyl-3-O-methyl-1-O-trimethylsilyl-cyclohexen-1,3,5-triol and its isomers;

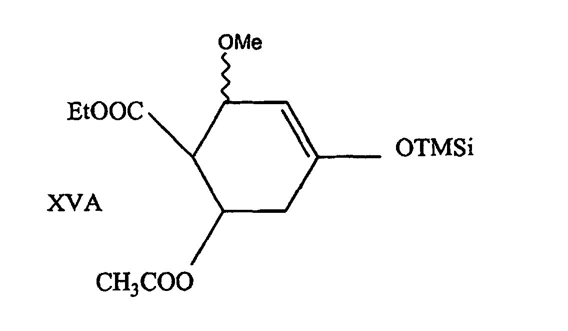

28. (±) 4-hydroxymethyl-cyclohex-2-en-1,5-diol.

29. (1*R*, 4*R*, 5*S*)-4-hydroxymethyl-cyclohex-2-en-1,5-diol.

30. (1*S*, 4*S*, 5*R*)-4-hydroxymethyl-cyclohex-2-en-1,5-diol.

31. (±) 5,7-*O*-benzylidene-4-hydroxymethylcyclohex-2-en-1,5-diol.

32. (1*R*, 4*R*, 5*S*)-5,7-*O*-benzylidene-4-hydroxymethyl-cyclohex-2-en-1,5-diol.

33. (1*S*, 4*S*, 5*R*)-5,7-*O*-benzylidene-4-hydroxymethyl-cyclohex-2-en-1,5-diol.

34. Compound according to any of the preceding claims 24-26 selected from the following group:
- (4*S*,5*R*)-5-Benzoyloxy-4-benzoyloxymethyl-cyclohex-2-en-1-one,
- (1S,4*S*,5*R*)-5-Benzoyloxy-4-benzoyloxymethyl-cyclohex-2-en-1-ol,
- (4*R*,5*S*)-4-*tert*-Butyldimethylsilyloxymethyl-5-tert-butyldimethylsilyloxy-cyclohex-2-en-1-one,
- (1R,4*R*,5*S*)-5-(tert-Butyldimethylsilyloxy)-4-(tert-butyldimethylsilyloxymethyl)-cyclohex-2-en-1-ol.

35. Pharmaceutical composition comprising a compound according to any of the claims 1-14, 24-34.

36. A pharmaceutical composition as claimed in any of the claims 1-14, 24-34, having antiviral activity towards herpetic viruses selected but not limited from the group consisting of herpes simplex virus type 1 (HSV-1), herpes simplex virus type 2 (HSV-2), Varicella zoster virus (VZV), and cytomegalovirus (CMV), as well as towards pox viruses, e.g. vaccinia virus (VV).

37. A pharmaceutical composition as claimed in claim 36 comprising said active ingredient in a concentration ranging from about 0.1-100 % by weight.

38. A pharmaceutical composition as claimed in claim 37, having the form which is selected from the group consisting of powders, suspensions, solutions, sprays, emulsions, unguents and creams.

39. Compound according to claim 1-14, 24-34 for use as an anti-viral agent.

40. Compound according to claim 1-14, 24-34 for use as an agent having anti-viral activity towards herpes viruses, pox viruses and relates viruses.

41. A pharmaceutical composition according to claim 35-38 for use as an anti-viral agent.

42. A pharmaceutical composition according to claim 35-38 for use as an agent having anti-viral activity towards herpetic viruses selected from the group comprising herpes simplex virus type 1 (HSV-1), herpes simplex virus type 2 (HSV-2), Varicella zoster virus (VZV), and cytomegalovirus (CMV), as well as towards pox viruses, e.g. vaccinia virus (VV), herpes viruses, pox viruses and related viruses.

## Patentansprüche

1. Sechsgliedrige, zumindest teilweise ungesättigte, carbozyklische Nukleosidverbindung, einschließlich des (-) Enantiomers, des (+) Enantiomers und pharmazeutisch akzeptabler Salze und Ester hiervon, wobei die Verbindungen durch die allgemeine Formel I repräsentiert werden: 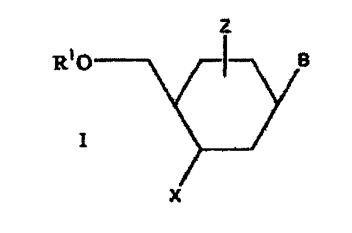 wobei:
- Z das Vorhandensein von ein oder mehreren Doppelbindungen im sechsgliedrigen carbozyklischen Ring repräsentiert,
- B ein heterozyklischer Ring ist, ausgewählt aus der Gruppe, die aus Pyrimidin- und Purin-Basen besteht,
- X Wasserstoff, Azido, F oder OR² ist,
- R¹ und R² gleich oder verschieden sind und dieselben oder verschiedene Schutzgruppen, Wasserstoff-, Alkyl-, Alkenyl-, Acyl- oder Phosphat-Komponenten repräsentieren, wobei;
- die Alkyl-Komponente ein gesättigtes, substituiertes oder unsubstituiertes geradkettiges oder verzweigtkettiges Kohlenwasserstoff-Radikal mit von 1-20, beispielsweise 1-16, 1-14, 1-12, 1-10, 1-8, 1-4 Kohlenstoffatomen ist,
- die Alkenyl-Komponente eine ungesättigte, der Alkyl-Gruppe artverwandte Komponente (Kongener) ist, und
- die Acyl-Komponente eine Alkanoyl- oder Aroyl-Komponente ist, wobei Alkanoyl ein Alkylcarbonyl-Radikal ist, wobei Alkyl wie oben beschrieben ist und Aroyl Benzoyl, substituiertes Benzoyl oder Naphtoyl repräsentiert.

2. Sechsgliedrige, zumindest teilweise ungesättigte, carbozyklische Nukleosidverbindung gemäß Anspruch 1, die eine Cyclylhexenylnukleosid-Verbindung mit der allgemeinen Formel II oder III ist:

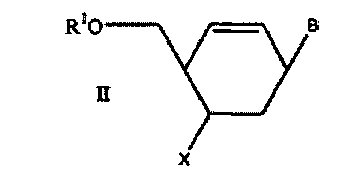

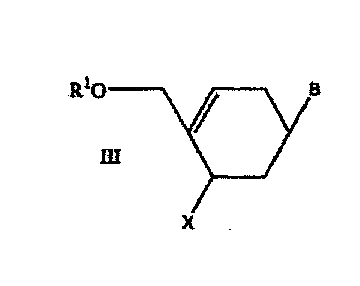

3. Verbindung nach Anspruch 1 oder 2, ausgewählt aus der folgenden Gruppe von Verbindungen, repräsentiert durch die Formeln IV-X':

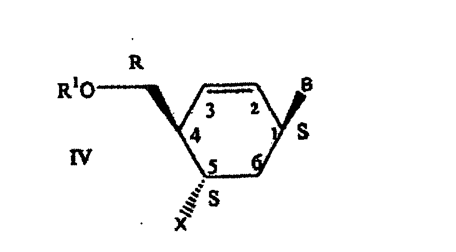

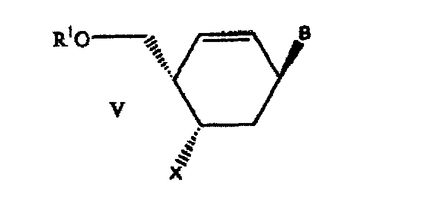

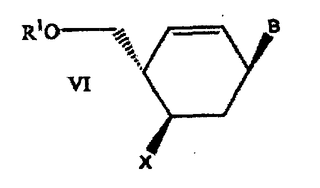

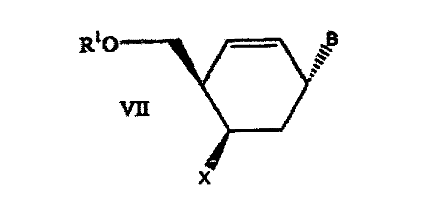

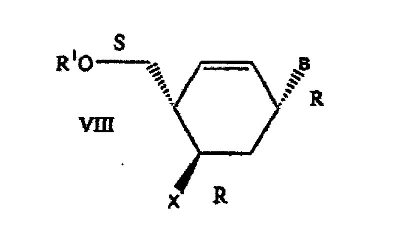

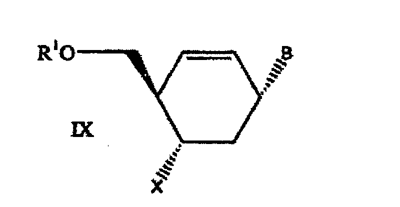

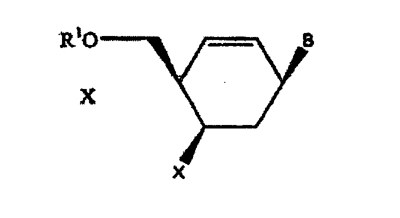

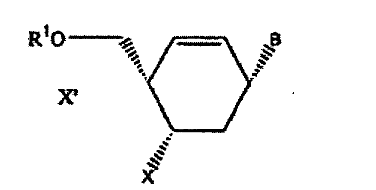

4. Verbindung nach einem der vorhergehenden Ansprüche, wobei der C₁-tragende B-Substituent und der C₅-tragende X-Substituent beide die (S)-Konfiguration aufweisen und der C₄-tragende-OR'-Substituent die (R)-Konfiguration aufweist, wie durch Formel IV in Anspruch 3 beschrieben ist.

5. Verbindung nach Anspruch 1, 2 oder 3, wobei der C₁-tragende B-Substituent und der C₅-tragende X-Substituent beide die (R)-Konfiguration aufweisen und der C₄-tragende-OR¹-Substituent die (S)-Konfiguration aufweist, wie durch Formel VIII in Anspruch 3 dargestellt ist.

6. Verbindung nach einem der Ansprüche 1-4, wobei X durch eine Hydroxyl-Gruppe in der (S)-Konfiguration repräsentiert wird.

7. Verbindung nach einem der Ansprüche 1, 2, 3 und 5, wobei X Hydroxyl in der (R)-Konfiguration ist.

8. Verbindung nach einem der vorhergehenden Ansprüche, wobei B aus der Gruppe abgeleitet ist, die aus Pyrimidin-Basen besteht.

9. Verbindung nach Anspruch 7, wobei die Pyrimidin-Base die allgemeine Formel XI aufweist: 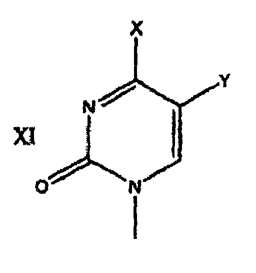 wobei X aus Folgendem ausgewählt ist;
- OH, NH₂, NHQ,
wobei;
- Q aus Folgendem ausgewählt ist;
OH oder C₁₋₅ Alkyl,
- Y aus Folgendem ausgewählt ist;
H, F, Cl, Br, I, C₁₋₅ Alkyl, Haloethyl oder CH=CH-R, wobei R Wasserstoff, Halogen oder C₁₋₅ Alkyl repräsentiert und wobei Haloethyl von 1 bis 4 F, Cl oder Br-Atome enthält.

10. Verbindung nach einem der vorhergehenden Ansprüche, wobei B aus der Gruppe ausgewählt ist, die aus substituiertem und unsubstituiertem Adenin, Guanin, 2,6-Diaminopurin, Hypoxanthin und Xanthin besteht.

11. Verbindung nach einem der vorhergehenden Ansprüche, wobei das B aus der Gruppe von Aza-, Deazä-, Desoxy- oder Desamino-Analoga der heterozyklischen Ringe, wie in einem der Ansprüche 8-10 definiert, ausgewählt ist.

12. Verbindung nach einem der vorhergehenden Ansprüche, wobei die Schutzgruppe eine Silyl-Schutzgruppe, vorzugsweise TBDMS, und/oder Benzoyl-Schutzgruppe und/oder eine C6H5-CH= Gruppe umfasst.

13. Verbindung nach einem der vorhergehenden Ansprüche 1-11, ausgewählt aus:
- 9-[(1S, 4R, 5S)-5-hydroxy-4-hydroxymethyl-2-cyclohexenyl] guanin
- 9-[(1R, 4S, 5R)-5-hydroxy-4-hydroxymethyl-2-cyclohexenyl].

14. Verbindung nach einem der Ansprüche 1-11, ausgewählt aus der folgenden Gruppe:
- 9-[(1S, 4R, 5S)-5-(tert-butyldimethylsilyloxy)-4-(tert-butyldimethylsilyloxymethyl)-2-cyclohexenyl] adenin
- 9-[(1S, 4R, 5S)-5-hydroxy-4-hydroxymethyl-2-cyclohexenyl] adenin
- 9-[(1S, 4R, 5S)-5-(tert-butyldimethylsilyloxy)-4-(tert-butyldimethylsilyloxymethyl)-2-cyclohexenyl]-2-amino-6-chlorpurin
- 9-[(1S, 4R, 5S)-5-hydroxy-4-hydroxymethyl-2-cyclohexenyl] guanin
- 9-[(1R, 4S, 5R)-5-benzoyloxy-4-benzoyloxymethyl-2-cyclohexenyl] adenin
- 9-[(1R, 4S, 5R)-5-hydroxy-4-hydroxymethyl-2-cyclohexenyl] adenin
- 9-[(1R, 4S, 5R)-5-benzoyloxy-4-benzoyloxymethyl-2-cyclohexenyl] guanin
- 9-[(1R, 4S, 5R)-5-hydroxy-4-hydroxymethyl-2-cyclohexenyl] guanin

15. Verfahren zur Bereitstellung einer Verbindung, des (-) Enantiomers, des (+) Enantiomers und pharmazeutisch akzeptabler Salze und Ester hiervon, gemäß einem der vorhergehenden Ansprüche, wobei das Verfahren die Schritte umfasst:
- eine Cyclohexenyl-Verbindung der allgemeinen Formel XII bereitzustellen; 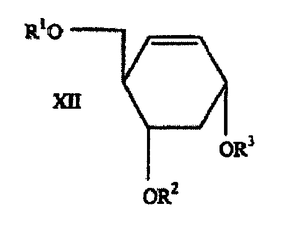
- wobei R¹ und R² Schutzgruppen sind und R³ eine Abgangsgruppe oder ein Wasserstoffatom ist, gefolgt von dem Schritt, die OR³-Gruppe durch eine Pyrimidin- oder Purin-Base zu ersetzen.

16. Verfahren nach Anspruch 15, wobei R³ Wasserstoff ist und wobei eine Reaktion vom Mitsunobo-Typ verwendet wird.

17. Verfahren nach Anspruch 15, wobei R³ eine Abgangsgruppe ist, die eine nukleophile Substitution ermöglicht.

18. Verfahren nach einem der vorhergehenden Ansprüche 15-17, wobei die Verbindung der allgemeinen Formel XII die chemische Formel XIII aufweist; 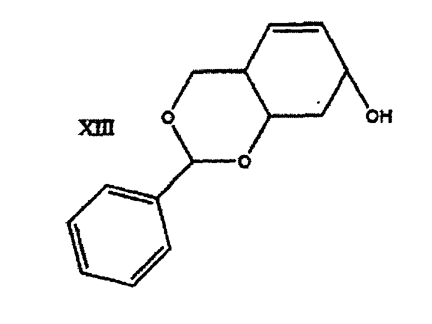 oder Analoga hiervon, entweder in einer Racematform oder als getrennte Isomere hiervon.

19. Verfahren nach einem der vorhergehenden Ansprüche 15-18, wobei die Verbindung XIII durch Umsetzen eines (±)4-Hydroxymethyl-cyclohex-2-en-1,5 diols der Formel XIV; 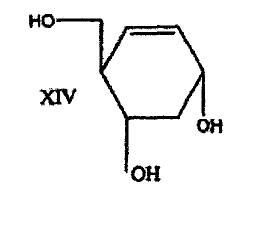 mit einem Benzaldehyd-Analogon und einer Lewis-Säure, vorzugsweise einem Benzyldehyddialkylacetal, am meisten bevorzugt Dimethylacetal, und p-Toluolsulfonsäure, bereitgestellt wird.

20. Verfahren nach einem der vorhergehenden Ansprüche 15-19, wobei die Verbindung XIV durch Reduktion einer Verbindung XVA oder XVB bereitgestellt wird, vorzugsweise unter Verwendung von Lithiumaluminiumhydrid oder einem Äquivalent hiervon als Reduktionsmittel; 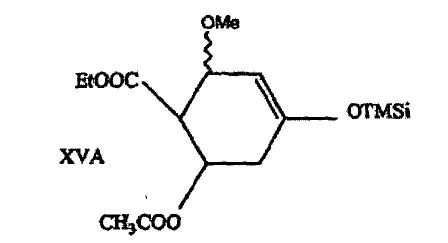 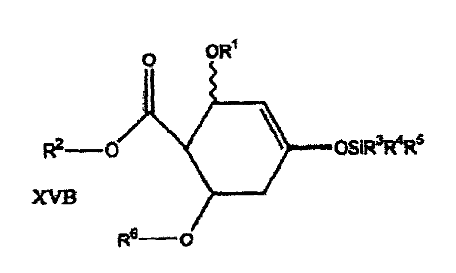 wobei für XVB:
R¹ und R² Alkyl- oder Alkenyl-Komponenten sind,
wobei:
- R¹ und R² gleich oder verschieden sind, und
- Alkyl ein gesättigtes, substituiertes oder unsubstituiertes Kohlenwasserstoffradikal mit von 1-20, beispielsweise 1-16, 1-14, 1-12, 1-10, 1-8, 1-4 Kohlenstoffatomen ist, das geradoder verzweigtkettig ist, und
- Alkenyl der ungesättigte Kongener der Alkyl-Gruppe ist, und
R³, R⁴ und R⁵ Alkyl-, Alkenyl- oder Aryl-Komponenten sind, wobei:
- R³, R⁴ und R⁵ gleich oder verschieden sind, und
- Alkyl ein gesättigtes, substituiertes oder unsubstituiertes geradkettiges oder verzweigtkettiges Kohlenwasserstoffradikal mit von 1-20, beispielsweise 1-16, 1-14, 1-12, 1-10, 1-8, 1-4 Kohlenstoffatomen ist und
- Alkenyl der ungesättigte Kongener der Alkyl-Gruppe ist, und
- Aryl, Phenyl oder substituiertes Phenyl repräsentiert, und
R⁶ eine Alkyl-, Alkenyl- oder Acyl-Komponente ist, wobei:
- Alkyl ein gesättigtes, substituiertes oder unsubstituiertes, geradkettiges oder verzweigtkettiges Kohlenwasserstoffradikal mit von 1-20, beispielweise 1-16, 1-14, 1-12, 1-10, 1-8, 1-4 Kohlenstoffatomen ist.
- Alkenyl der ungesättigte Kongener der Alkyl-Gruppe ist, und
- Acyl eine Alkanoyl- oder Aroyl-Komponente ist, wobei Alkanoyl ein Alkyl-Carbonyl-Radikal ist, wobei Alkyl wie oben beschrieben ist und Aroyl Benzoyl, substituiertes Benzoyl oder Naphtoyl repräsentiert.

21. Verfahren nach Anspruch 20, wobei Verbindung XVA oder XVB durch eine Diels-Alder-Reaktion erzeugt wird, durch die Zykloaddition eines geeigneten Diens und Dienophils, wobei vorzugsweise das Dien und Dienophil zusammen in Gegenwart eines Hydrochinons erhitzt werden.

22. Verfahren nach Anspruch 21, wobei das Dien die folgende chemische Struktur XVI aufweist und das Dienophil die folgende chemische Struktur XVII aufweist, wobei R¹, R², R³, R⁴, R⁵ und R⁶ wie in Anspruch 20 definiert sind;

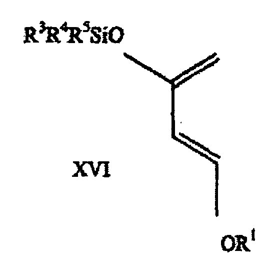

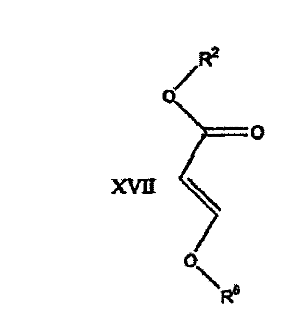

23. Verfahren nach Anspruch 22, wobei das Dien die chemische Struktur XVI' aufweist und das Dienophil die chemische Struktur XVIII aufweist;

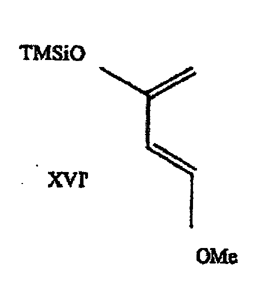

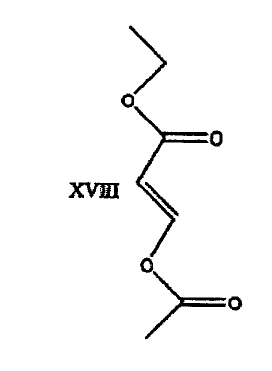

24. Sechsgliedrige, zumindest teilweise ungesättigte, carbozyklische Nukleosidverbindung, einschließlich des (-) Enantiomers, des (+) Enantiomers und pharmazeutisch akzeptabler Salze und Ester hiervon, wobei die Verbindungen durch die allgemeine Formel XII oder XIX repräsentiert werden; 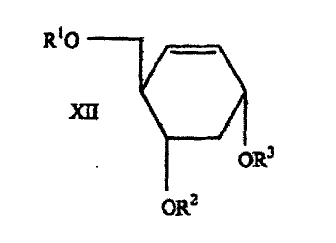 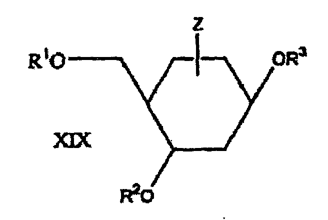 wobei:
- Z das Vorhandensein von ein oder mehreren Doppelbindungen im carbozyklischen Ring repräsentiert;
- R¹ und R² Schutzgruppen sind und R³ eine Abgangsgruppe oder ein Wasserstoffatom ist.

25. Verbindung nach Anspruch 24, wobei;
R¹ und R² gleich oder verschieden sind und Wasserstoff, Alkyl-, Alkenyl-, Acyl- oder Phosphat-Komponenten repräsentieren, oder R¹ und R² eine zyklische Schutzgruppe repräsentieren; wobei:
- Alkyl ein gesättigtes, substituiertes oder unsubstituiertes geradkettiges oder verzweigtkettiges Kohlenwasserstoff-Radikal mit von 1-20, beispielsweise 1-16, 1-14, 1-12, 1-10, 1-8, 1-4 Kohlenstoffatomen ist; und
- Alkenyl der ungesättigte Kongener der Alkyl-Gruppe ist, und
- Acyl eine Alkanoyl- oder Aroyl-Komponente ist, wobei Alkanoyl ein Alkylcarbonyl-Radikal ist, wobei Alkyl wie oben beschrieben ist und Aroyl Benzoyl, substituiertes Benzoyl oder Naphtoyl repräsentiert; und
R³ eine Wasserstoff-, eine Alkylsulfonyl- oder eine Arylsulfonyl-Komponente repräsentiert, wobei:
- Alkyl ein gesättigtes, substituiertes oder unsubstituiertes Kohlenwasserstoff-Radikal mit von 1-6 Kohlenstoffatomen und gerad- oder verzweigtkettig ist, und
- Aryl Phenyl oder substituiertes Phenyl repräsentiert.

26. Cyclohexenyl-Verbindung, einschließlich des (-) Enantiomers, des (+) Enantiomers und pharmazeutisch akzeptabler Salze und Ester hiervon, wobei die Verbindung durch die allgemeine Formel XVB repräsentiert wird; 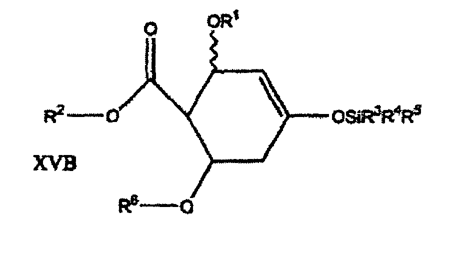 wobei R¹ und R² Alkyl- oder Alkenyl-Komponenten sind, wobei:
- R¹ oder R² gleich oder verschieden sind, und
- Alkyl ein gesättigtes, substituiertes oder unsubstituiertes geradkettiges oder verzweigtkettiges Kohlenwasserstoff-Radikal mit von 1-20, beispielsweise 1-16, 1-14, 1-12, 1-10, 1-8, 1-4 Kohlenstoffatomen ist,
- Alkenyl der ungesättigte Kongener der Alkyl-Gruppe ist, und
R³, R⁴ und R⁵ Alkyl-, Alkenyl- oder Aryl-Komponenten sind, wobei:
- R³, R⁴ und R⁵ gleich oder verschieden sind, und
- Alkyl ein gesättigtes, substituiertes oder unsubstituiertes gerad- oder verzweigtkettiges Kohlenwasserstoff-Radikal mit von 1-20, beispielsweise 1-16, 1-14, 1-12, 1-10, 1-8, 1-4 Kohlenstoffatomen ist; und
- Alkenyl der ungesättigte Kongener der Alkyl-Gruppe ist, und
- Aryl Phenyl oder substituiertes Phenyl repräsentiert, und
R⁶ eine Alkyl-, Alkenyl- oder Acyl-Komponente ist, wobei:
- Alkyl ein gesättigtes, substituiertes oder unsubstituiertes geradkettiges oder verzweigtkettiges Kohlenwasserstoff-Radikal mit von 1-20, beispielsweise 1-16, 1-14, 1-12, 1-10, 1-8, 1-4 Kohlenstoffatomen ist, und
- Alkenyl der ungesättigte Kongener der Alkyl-Gruppe ist, und
- Acyl eine Alkanoyl- oder Aroyl-Komponente ist, wobei Alkanoyl ein Alkylcarbonyl-Radikal ist, wobei Alkyl wie oben beschrieben ist und Aroyl Benzoyl, substituiertes Benzoyl oder Naphtoyl repräsentiert.

27. Verbindung nach Anspruch 26, die die Formel XVA aufweist; 5-O-Acetyl-4-ethoxycarbonyl-3-O-methyl-1-O-trimethylsilyl-cyclohexen-1,3,5-triol und deren Isomere;

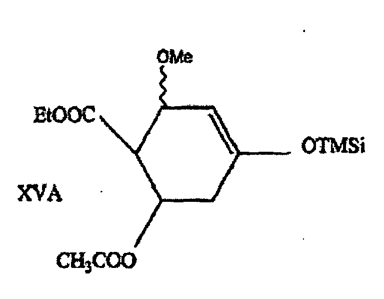

28. (±) 4-Hydroxymethyl-cyclohex-2-en-1,5-diol.

29. (1R, 4R, 5S)-4-hydroxymethyl-cyclohex-2-en-1,5-diol.

30. (1S, 4S, 5R)-4-hydroxymethyl-cyclohex-2-en-1,5-diol.

31. (±) 5,7-O-Benzyliden-4-hydroxymethyl-cyclohex-2-en-1,5-diol.

32. (1R, 4R, 5S)-5,7-O-benzyliden-4-hydroxymethyl-cyclohex-2-en-1,5-diol.

33. (1S, 4S, 5R)-5,7-O-Benzyliden-4-hydroxymethyl-cyclohex-2-en-1,5-diol.

34. Verbindung nach einem der vorhergehenden Ansprüche 24-26, ausgewählt aus der folgenden Gruppe:
- (4S, 5R)-5-Benzoyloxy-4-benzoyloxymethyl-cyclohex-2-en-1-on,
- (1S, 4S, 5R)-5-Benzoyloxy-4-benzoyloxymethyl-cyclohex-2-en-1-ol,
- (4R, 5S)-4-tert-Butyldimethylsilyloxymethyl-5-tert-butyldimethylsilyloxy-cyclohex-2-en-1-on,
- (1R, 4R, 5S)-5-(tert-Butyldimethylsilyloxy)-4-(tert-butyldimethylsilyloxymethyl)-cyclohex-2-en-1-ol.

35. Pharmazeutische Zusammensetzung, die eine Verbindung nach einem der Ansprüche 1-14, 24-34 umfasst.

36. Pharmazeutische Zusammensetzung nach einem der Ansprüche 1-14, 24-34 mit einer antiviralen Wirksamkeit gegenüber Herpes-Viren, ausgewählt, jedoch nicht beschränkt auf die Gruppe, die aus Herpes simplex Virus Typ 1 (HSV-1), Herpes simplex Virus Typ 2 (HSV-2), Varicella zoster Virus (VZV) und Zytomegalie-Virus (CMV) besteht, ebenso wie gegenüber Pocken-Viren, beispielsweise Vaccinia-Viren (Vaccinia-Virus = VV).

37. Pharmazeutische Zusammensetzung nach Anspruch 36, die einen aktiven bzw. wirksamen Inhaltsstoff in einem Konzentrationsbereich von ungefähr 0,1 bis 100 Gew.-% umfasst.

38. Pharmazeutische Zusammensetzung nach Anspruch 37, die eine Form aufweist, ausgewählt aus der Gruppe, die aus Pulvern, Suspensionen, Lösungen, Sprays, Emulsionen, Salben und Cremes besteht.

39. Verbindung nach Anspruch 1-14, 24-34 zur Verwendung als antivirales Mittel.

40. Verbindung nach Anspruch 1-14, 24-34 zur Verwendung als Mittel mit einer antiviralen Aktivität gegenüber Herpes-Viren, Pocken-Viren und verwandten Viren.

41. Pharmazeutische Zusammensetzung nach Anspruch 35-38 zur Verwendung als antivirales Mittel.

42. Pharmazeutische Zusammensetzung nach Anspruch 35-38 zur Verwendung als Mittel mit antiviraler Aktivität gegenüber Herpes-Viren, ausgewählt aus der Gruppe, die Herpessimplex Virus Typ 1 (HSV-1), Herpes simplex Virus-Typ 2 (HSV-2), Varicella zoster Virus (VZV) und das Zytomegalie-Virus (CMV) umfasst, ebenso wie gegenüber Pocken-Viren, beispielsweise Vaccinia-Viren (VV), Herpes-Viren, Pocken-Viren und verwandten Viren.

## Revendications

1. Composé nucléoside carbocyclique à six chaînons, au moins partiellement insaturé, comprenant l'énantiomère (-), l'énantiomère (+) et les sels et les esters pharmaceutiquement acceptables de celui-ci, les composés représentés par la formule générale I : 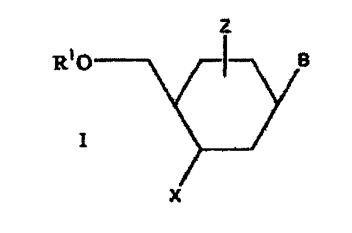 dans laquelle :
- Z représente la présence d'une ou plusieurs doubles liaisons dans le noyau carbocyclique à six chaînons,
- B est un noyau hétérocyclique choisi dans le groupe constitue par les bases pyrimidine et purine,
- X est un atome d'hydrogène, un groupe azido, F ou OR³,
- R¹ et R² sont identiques ou différents et représentent des groupes protecteurs identiques ou différents, un atome d'hydrogène, des fractions alkyle, alcényle, alcyle ou phosphate dans lesquelles :
- la fraction alkyle est un radical hydrocarboné saturé, substitué ou non substitué, à chaîne droite ou ramifiée comportant de 1 à 20, par exemple de 1 à 16, de 1 à 14, de 1 à 12, de 1 à 10, de 1 à 8, de 1 à 4, atomes de carbone,
- la fraction alcényle est un congénère insaturé du groupe alkyle et,
- la fraction alcyle est une fraction alcanoyle ou aroyle, dans laquelle la fraction alcanoyle est un radical alkylcarbonyle, dans lequel alkyle est tel que décrit ci-dessus et aroyle représente le benzoyle, un benzoyle substitué ou le naphtoyle.

2. Composé nucléoside carbocyclique à six chaînons, au moins partiellement insaturé, selon la revendication 1, étant un composé nucléoside cyclohexényle répondant à la formule générale II ou III.

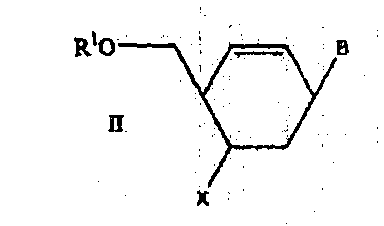

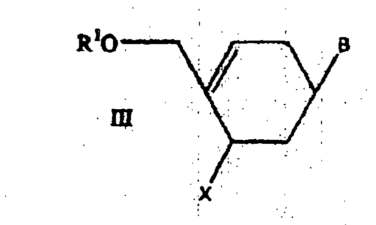

3. Composé selon les revendications 1 ou 2, choisi dans le groupe suivant de composés représentés par les formules IV à X' :

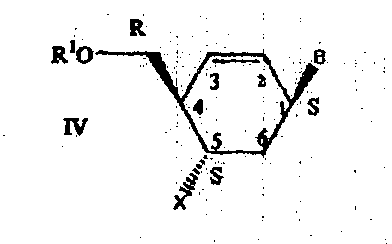

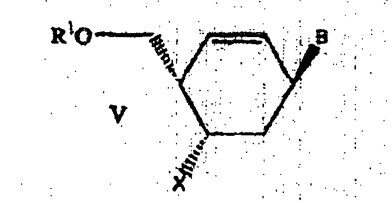

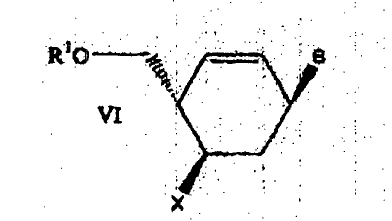

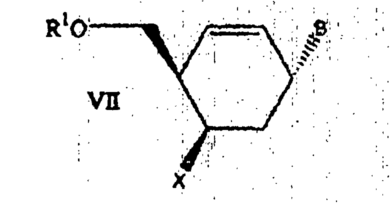

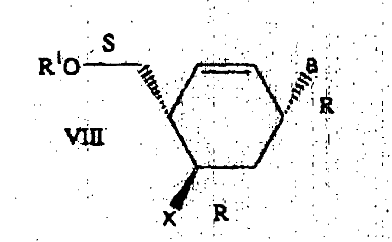

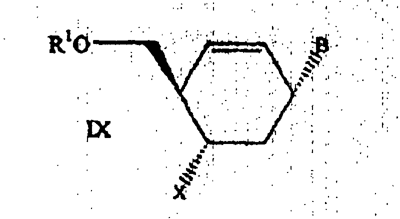

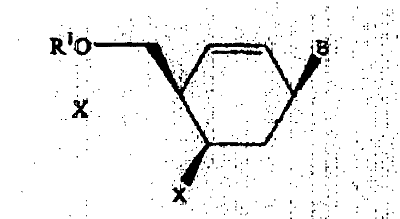

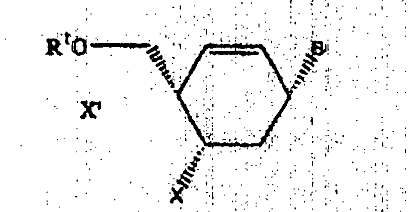

4. Composé selon l'une quelconque des revendications précédentes, dans lequel le C₁ portant le substituant B et le C₅ portant le substituant X ont tous deux la configuration (S), et le C₄ portant le substituant -OR¹ a la configuration (R), tel que représenté par la formule IV dans la revendication 3.

5. Composé selon les revendications 1, 2 ou 3, dans lequel le C₁ portant le substituant B et le C₅ portant le substituant X ont tous deux la configuration (R), et le C₄ portant le substituant -OR¹ a la configuration (S), tel que représenté par la formule VII dans la revendication 3.

6. Composé selon l'une quelconque des revendications 1 à 4, dans lequel X est représenté par un groupe hydroxyle dans la configuration (S).

7. Composé selon l'une quelconque des revendications 1, 2, 3 et 5, dans lequel X est un groupe hydroxyle dans la configuration (R).

8. Composé selon l'une quelconque des revendications précédentes, dans lequel B est dérivé du groupe constitué par les bases pyrimidine.

9. Composé selon la revendication 7, dans lequel la base pyrimidine répond à la formule générale XI : 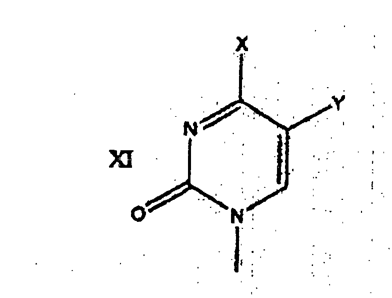 dans laquelle X est choisi parmi ce qui suit :
- OH, NH₂, NHQ,
dans lequel:
- Q est choisi parmi ce qui suit :
OH ou un groupe alkyle en C₁ à C₅,
- Y est choisi parmi ce qui suit :
H, F, Cl, Br, I, un groupe alkyle en C₁ à C₅, halogénoéthyle ou CH=CH-R, dans lequel R représente un atome d'hydrogène, d'halogène, ou un groupe alkyle en C₁ à C₅, et dans lequel l'halogénoéthyle contient de 1 à 4 atomes de F, Cl ou Br.

10. Composé selon l'une quelconque des revendications précédentes, dans lequel B est choisi dans le groupe constitué par l'adénine, la guanine, la 2,6-diaminopurine, l'hypoxanthine et la xanthine substituées et non substituées.

11. Composé selon l'une quelconque des revendications précédentes, dans lequel B est choisi dans le groupe des analogues aza, déaza déoxy ou déamino des noyaux hétérocycliques, tels que définis dans l'une quelconque des revendications 8 à 10.

12. Composé selon l'une quelconque des revendications précédentes, dans lequel le groupe protecteur comprend un groupe protecteur silyle, de préférence le TBDMS, et/ou un groupe protecteur benzoyle et/ou un groupe C₆ H₅-CH=.

13. Composé selon l'une quelconque des revendications précédentes 1 à 11, choisi parmi :
- la 9-[(1S,4R,5S)-5-hydroxy-4-hydroxyméthyl-2-cyclohexényl]-guanine
- la 9-[1R,4S,5R)-5-hydroxy-4-hydroxyméthyl-2-cyclohexényl] guanine.

14. Composé selon l'une quelconque des revendications 1 à 11, choisi dans le groupe suivant :
- la 9-[(1S,4R,5S)-5-(tert-butyldiméthylsilyloxy)-4-(tert-butyl-diméthylsilyloxyméthyl)-2-cyclo-hexényl]adénine
- la 9-[(1S,4R,5S)-5-hydroxy-4-hydroxyméthyl-2-cyclohexényl]-adénine
- la 9-[(1S,4R,5S)-5-(tert-butyldiméthylsilyloxy)-4-(tert-butyldiméthylsilyloxyméthyl)-2-cyclohexényl]-2-amino-6-chloropurine
- la 9-[(1S,4R,5S)-5-hydroxy-4-hydroxyméthyl-2-cyclohexényl]guanine
- la 9-[(1R,4S,5R)-5-benzoyloxy-4-benzoyloxyméthyl-2-cyclohexényl]adénine
- la 9-[(1R,4S,5R)-5-hydroxy-4-hydroxyméthyl-2-cyclohexényladénine
- la 9-[(1R,4S,5R)-5-benzoyloxy-4-benzoyloxyméthyl-2-cyclohexényl]guanine
- la 9-[(1R,4S,5R)-5-hydroxy-4-hydroxyméthyl-2-cyclohexényl]-guanine

15. Procédé pour l'obtention d'un composé, l'énantiomère (-), l'énantiomère (+) et les sels et esters pharmaceutiquement acceptables de celui-ci selon l'une quelconque des revendications précédentes, ledit procédé comprenant les étapes consistant à :
- former un composé cyclohexényle de formule générale XII : 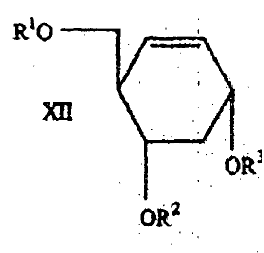
dans laquelle R¹ et R² sont des groupes protecteurs et R³ est un groupe partant ou un atome d'hydrogène, suivie par l'étape consistant à substituer le groupe -OR³ par une base pyrimidine ou purine.

16. Procédé selon la revendication 15, dans lequel R³ est un atome d'hydrogène et dans lequel une réaction de type Mitsunobo est utilisée.

17. Procédé selon la revendication 15, dans lequel R³ est un groupe partant permettant une substitution nucléophite.

18. Procédé selon l'une quelconque des revendications précédentes 15 à 17, dans lequel le composé de formule générale XII répond à la formule chimique XIII : 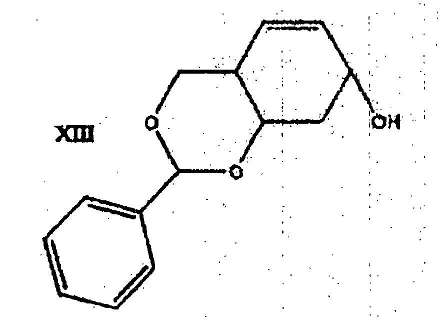 ou les analogues de celui-ci soit sous forme de racemate, soit les isomères séparés de ceux-ci.

19. Procédé selon l'une quelconque des revendications précédentes 15 à 18, dans lequel le composé XIII est formé par réaction du (±) 4-hydroxyméthyl-cyclohex-2-en-1,5 diol de formule XIV : 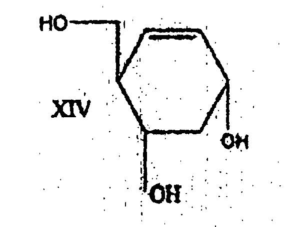 avec un analogue benzaldéhyde et un acide de lewis, étant de préférence un benzaldéhyde dialkylacétal, de manière la plus préférée le diméthylacétal, et l'acide p-toluènesulfonique.

20. Procédé selon l'une quelconque des revendications précédentes 15 à 19, dans lequel le composé XIV est formé par la réduction du composé XVA ou XVB, en utilisant de préférence de l'hydrure de lithium aluminium ou un équivalent de celui-ci en tant qu'agent réducteur ; 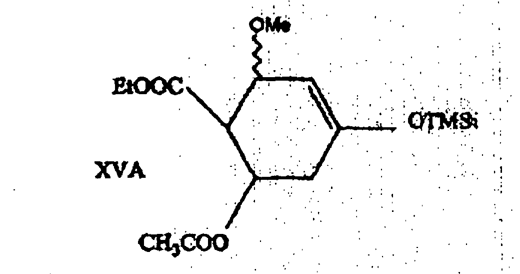 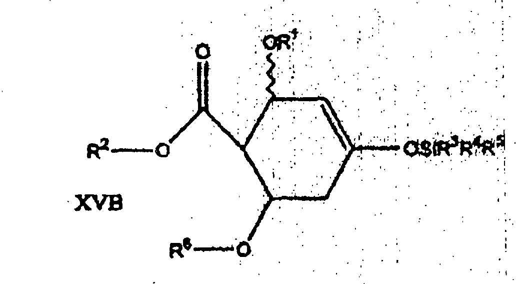 dans lesquelles pour XVB :
R¹ et R² sont des fractions alkyle ou alcényle, dans lesquelles :
- R¹ et R² sont identiques ou différents, et
- alkyle est un radical hydrocarboné saturé substitué ou non substitué comportant de 1 à 20, par exemple, de 1 à 16, de 1 à 14, de 1 à 12, de 1 à 10, de 1 à 8, de 1 à 4, atomes de carbone et étant à chaîne droite ou ramifiée, et
- alcényle est le congénère insaturé du groupe alkyle, et
R³, R⁴ et R⁵ sont des fractions alkyle, alcényle ou aryle, dans lesquelles :
- R³, R⁴ et R⁵ sont identiques ou différents, et
- alkyle est un radical hydrocarboné saturé substitué ou non substitué à chaîne droite ou ramifiée comportant de 1 à 20, par exemple, de 1 à 16, de 1 à 14, de 1 à 12, de 1 à 10, de 1 à 8, de 1 à 4, atomes de carbone et
- alcényle est le congénère insaturé du groupe alkyle, et
- aryle représente le phényle ou un phényle substitué, et
R⁶ est une fraction alkyle, alcényle ou acyle, dans laquelle :
- alkyle est un radical hydrocarboné saturé substitué ou non substitué à chaîne droite ou ramifiée comportant de 1 à 20, par exemple, de 1 à 16, de 1 à 14, de 1 à 12, de 1 à 10, de 1 à 8, de 1 à 4, atomes de carbone,
- alcényle est le congénère insaturé du groupe alkyle et
- acyle est une fraction alcanoyle ou aroyle, dans laquelle alcanoyle est un radical alkylcarbonyle, dans lequel alkyle est tel que décrit ci-dessus et aroyle représente le benzoyle, un benzoyle substitué ou le naphtoyle.

21. Procédé selon la revendication 20, dans lequel le composé XVA ou XVB est obtenu par une réaction de Diels-Alder, par la cyclo addition d'un diène approprié et d'un diénophile dans lequel, de préférence, le diène et le diénophile sont chauffés ensemble en présence d'hydroquinone.

22. Procédé selon la revendication 21, dans lequel le diène a la structure chimique XVI suivante, et le diénophile a la structure chimique XVII suivante, dans lesquelles R¹, R², R³, R⁴, R⁵ et R⁶ sont tels que définis dans la revendication 1 :

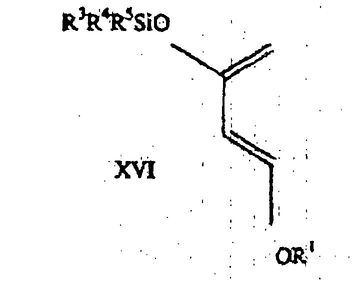

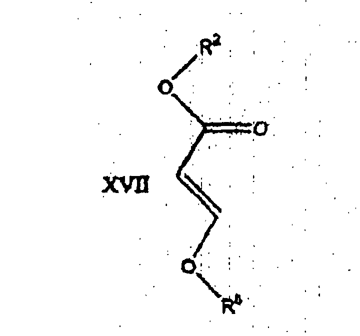

23. Procédé selon la revendication 22, dans lequel le diène a la structure chimique XVI' et le diénophile a la structure chimique XVIII :

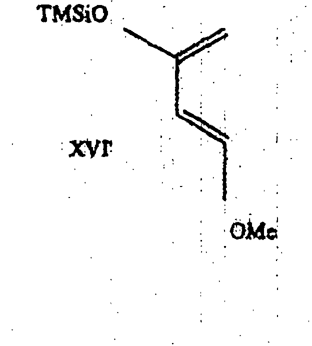

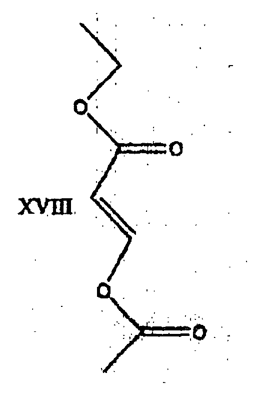

24. Composé nucléoside carbocyclique à six chaînons, au moins partiellement insaturé, comprenant l'énantiomère (-), l'énantiomère (+) et les sels et esters pharmaceutiquement acceptables de celui-ci, les composés représentés par la formule générale XII ou XIX : 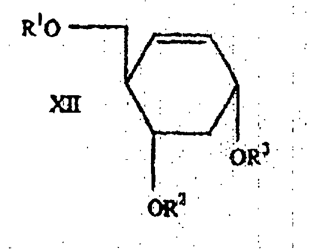 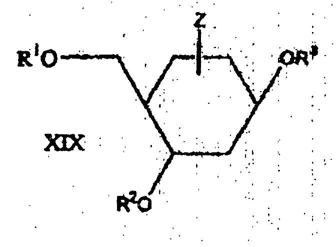 dans lesquelles :
- Z représente la présence d'une ou plusieurs doubles liaisons dans le noyau carbocyclique,
- R¹ et R² sont des groupes protecteurs et R³ est un groupe partant ou un atome d'hydrogène.

25. Composé selon la revendication 24, dans lequel
R¹ et R² sont identiques ou différents et un atome d'hydrogène des fractions alkyle, alcényle, alcyle ou phosphate sont représentées, ou R¹ et R² représentent un groupe protecteur cyclique, dans lequel :
- alkyle est un radical hydrocarboné saturé, substitué ou non substitué, à chaîne droite ou ramifiée, comportant de 1 à 20, par exemple de 1 à 16, de 1 à 14, de 1 à 12, de 1 à 10, de 1 à 8, de 1 à 4, atomes de carbone, et
- alcényle est le congénère insaturé du groupe alkyle, et
- acyle est une fraction alcanoyle ou aroyle, dans laquelle alcanoyle est un radical alkylcarbonyle, dans lequel alkyle est tel que décrit ci-dessus et aroyle représente le benzoyle, un benzoyle substitué ou le naphtoyle ; et
R³ représente un atome d'hydrogène, une fraction alkylsulfonyle ou une fraction arylsulfonyle, dans lesquelles :
- alkyle est un radical hydrocarboné saturé substitué ou non substitué comportant de 1 à 6 atomes de carbone et une chaîne droite ou ramifiée, et
- aryle représente le phényle ou un phényle substitué.

26. Composé cyclohexényle, comprenant l'énantiomère (-), l'énantiomère (+) et les sels et esters pharmaceutiquement acceptables de celui-ci, le composé représenté par la formule générale XVB : 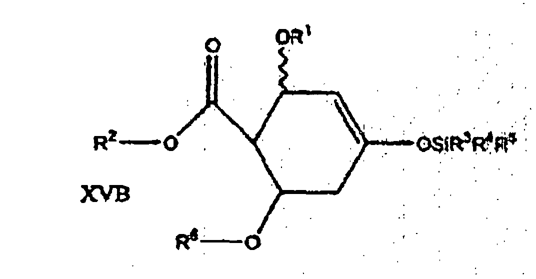 dans laquelle R¹ et R² sont des fractions alkyle ou alcényle, dans lesquelles :
- R¹ et R² sont identiques ou différents, et
- alkyle est un radical hydrocarboné saturé substitué ou non substitué, à chaîne droite ou ramifiée, comportant de 1 à 20, par exemple, de 1 à 16, de 1 à 14, de 1 à 12, de 1 à 10, de 1 à 8, de 1 à 4 atomes de carbone,
- alcényle est le congénère insaturé du groupe alkyle, et
R³, R⁴ et R⁵ sont des fractions alkyle, alcényle ou aryle dans lesquelles :
- R³, R⁴ et R⁵ sont identiques ou différents, et
- alkyle est un radical hydrocarboné saturé, substitué ou non substitué, à chaîne droite ou ramifiée, comportant de 1 à 20, par exemple de 1 à 16, de 1 à 14, de 1 à 12, de 1 à 10, de 1 à 8, de 1 à 4 atomes de carbone et,
- alcényle est le congénère insaturé du groupe alkyle, et
- aryle représente le phényle ou un phényle substitué, et
R⁶ est une fraction alkyle, alcényle ou acyle, dans laquelle :
- alkyle est un radical hydrocarboné saturé substitué ou non substitué à chaîne droite ou ramifiée comportant de 1 à 20, par exemple de 1 à 16, de 1 à 14, de 1 à 12, de 1 à 10, de 1 à 8, de 1 à 4 atomes de carbone, et
- alcényle est le congénère insaturé du groupe alkyle, et
- acyle est une fraction alcanoyle ou aroyle, dans laquelle alcanoyle est un radical alkylcarbonyle, dans lequel alkyle est tel que décrit ci-dessus et aroyle représente le benzoyle, un benzoyle substitué ou le naphtoyle.

27. Composé selon la revendication 26, répondant à la formule XVA, étant le 5-O-acétyl-4-éthoxycarbonyl-3-O-méthyl-1-O-triméthylsilyl-cyclohexèn-1,3,5-triol et ses isomères ;

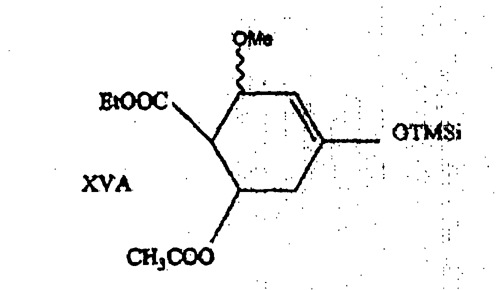

28. (±)4-hydroxyméthyl-cyclohex-2-èn-1,5-diol.

29. (1R,4R,5S)-4-hydroxyméthyl-cyclohex-2-èn-1,5-diol.

30. (1S,4S,5R)-4-hydroxyméthyl-cyclohex-2-èn-1,5-diol.

31. (±) 5,7-O-benzylidène-4-hydroxyméthyl-cylohex-2-èn-1,5-diol

32. (1R,4R,5S)-5,7-O-benzylidène-4-hydroxyméthyl-cyclohex-2-èn-1,5-diol.

33. (1S,4S,5R)-5,7-O-benzylidène-4-hydroxyméthyl-cyclohex-2-èn-1,5-diol.

34. Composé selon l'une quelconque des revendications précédentes 24 à 26, choisi dans le groupe suivant :
- la (4S,5R)-5-benzoyloxy-4-benzoyloxyméthyl-cyclohex-2-èn-1-one,
- le (1S,4S,5R)-5-benzoyloxy-4-benzoyloxyméthyl-cyclohex-2-èn-1-ol,
- la (4R,5S)-4-tert-butyldiméthylsilyloxyméthyl-5-tert-butyldiméthylsilyloxy-cyclohex-2-èn-1-one,
- le (1R,4R,5S)-5-(tert-butyldiméthylsilyloxy)-4-(tert-butyldiméthylsilyloxyméthyl)-cyclohex-2-èn-1-ol.

35. Composition pharmaceutique comprenant un composé selon l'une quelconque des revendications 1 à 14, 24 à 34.

36. Composition pharmaceutique selon l'une quelconque des revendications 1 à 14, 24 à 34, ayant une activité antivirale envers les virus herpétiques choisis mais non limités dans le groupe constitué par l'herpès simplex virus type 1 (HSV-1), l'herpès simplex virus type 2 (HSV-2), le virus varicelle-zona (VZV) et le virus cytomégalique (CMV), de même qu'envers les poxvirus, par exemple le virus de la vaccine (VV).

37. Composition pharmaceutique selon la revendication 36, comprenant ledit ingrédient actif en une concentration variant d'environ 0,1 à 100 % en poids.

38. Composition pharmaceutique selon la revendication 37, ayant la forme qui est choisie dans le groupe constitué par les poudres, les suspensions, les solutions, les pulvérisations, les émulsions les pommades et les crèmes.

39. Composé selon la revendication 1 à 14, 24 à 34 pour utilisation en tant qu'agent antiviral.

40. Composition selon la revendication 1 à 14, 24 à 34 pour utilisation en tant qu'agent ayant une activité antivirale envers les virus herpétiques, les poxvirus et les virus apparentés.

41. Composition pharmaceutique selon la revendication 35 à 38 pour utilisation en tant qu'agent antiviral.

42. Composition pharmaceutique selon la revendication 35 à 38 pour utilisation en tant qu'agent ayant une activité antivirale envers les virus herpétiques choisis dans le groupe comprenant herpès simplex virus type 1 (HSV-1), l'herpès simplex virus type 2 (HSV-2), le virus varicelle-zona (VZV) et le virus cytomégalique (CMV), de même qu'envers les poxvirus, par exemple le virus de la vaccine (VV), les virus herpétiques, les poxvirus et les virus apparentés.
